# EUROPEAN PATENT APPLICATION

(11) **EP 2 905 277 A1**
(43) Date of publication of application: **12.08.2015**
(21) Application number: 14154323.1
(22) Date of filing: 07.02.2014
(51) Int. Cl.: C07D 209/58, H01L 51/00

(54) **1',2',5'-trisubstituted Fulleropyrrolidines**

(71) Applicant: LANXESS Deutschland GmbH, 50569 Köln (DE); Institute of Problems of Chemical Physics RAS, Chernogolovka 142432, Moscow Region (RU)
(72) Inventor: Mumyatov, Alexander, V., Chernogolovka, Moscow region,142432 (RU); Mukhacheva, Olga A., Chernogolovka, Moscow region,142432 (RU); Novikov, Dmitry V., Dr., Chernogolovka, Moscow region,142432 (RU); Prudnov, Fedor A., Chernogolovka, Moscow region,142432 (RU); Troshin, Pavel A., Dr., Chernogolovka, Moscow region,142432 (RU)
(74) Representative: Wichmann, Birgid

(57) **Abstract**

The present invention relates to the use of 1',2',5'-trisubstituted fulleropyrrolidines of formula (1) and their use in optoelectronic devices, preferably in photovoltaic cells, preferably in organic photovoltaic cells to improve the open circuit voltage and energy conversion efficiency of solar cells.

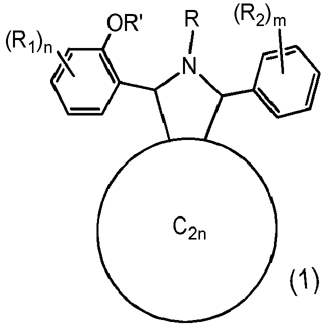

## Description

The present invention relates to 1',2',5'-trisubstituted fulleropyrrolidines an their use in photovoltaic cells, preferably in organic photovoltaic cells, especially preferred in organic bulk heterojunction solar cells and photodetectors to improve the photogenerated voltage and energy conversion efficiency of the devices.

Compounds of the present invention are fullerenopyrrolidines according to the CA nomenclature or pyrrolidinofullerenes according to the IUPAC nomenclature (see http://www.chem.qmul.ac.uk/iupac/fullerene/Fu07.html). The name "fulleropyrrolidine" is a trivial name given by M. Prato, used in all his publications, and adopted by many others and used in the present specification for fullerene derivatives according to the present invention.

Photovoltaic devices allow the most simple and efficient conversion of the solar energy to electricity. The first generation of solar cells based on crystalline silicon is known since the middle of the last century. However, wide scale distribution of such devices has been long time limited by their extremely high cost. The typical installation cost of solar cells based on crystalline silicon technologies stays in the range 2-3 US $ per every watt of energy generated at maximal (peak) solar irradiance, denoted as watt-peak, Wₚ. Organic solar cells are expected to be able for producing electricity at costs around 20 cents per Wₚ. This level might be approached by implementation of the devices yielding reasonably high power conversion efficiencies of 8-16% at very low module costs (40-60 US $/m²). Further improvements of organic solar cells in terms of performance, life-time, module design and production technologies might lead to a breakthrough in the renewable energy technology. At the end, the energy generated by solar light conversion should become cheaper than the energy that is currently produced by combustion of fossil fuels.

There are also many additional advantages of the organic thin film solar cells that can be illustrated as follows.
- Mechanical flexibility allows one to adapt them to any curved surfaces;
- Lightweight nature of thin film solar cells makes them ideal suiting for portable electronic applications;
- their integration into cloths (power-suite) and military canopies has been already demonstrated;
- High sensitivity at low light intensities allows indoor applications to collect scattered light, preferably for their use as decorative energy-generating wall-paper

Different examples of organic solar cells have entered the phase of commercialization recently. However, the market potential of organic solar cells is limited by their relatively low power conversion efficiency and short life times. Therefore, substantial improvements of the photoactive material combinations and the device architectures are required.

Use of fullerene derivatives with reduced electron affinity is one of the most promising ways for improving the performance of organic photovoltaic devices. The use of fullerene derivatives in photovoltaic cells was already described in DE 19 515 305 A1.

Science 1995, 270, 1789-1791 describes the use of [6,6]-phenyl-C₆₁-butyric acid methylester ([60]-PCBM, hereinafter also referred to as PCBM, to enhance the high power-conversion efficiencies in polymeric solar cells.

The use of Bis-[70]-PCBM and Bis-[60]-PCBM having reduced electron affinity in photovoltaic cells is described in US 2010/0224252 A1. Adv. Mater., 2008, 20, 2116 describes the use of bis-PCBM adducts with reduced electron affinity for enhancing the electrical performance of organic solar cells.

WO 2009/086210 A2, Am. Chem. Soc. 2010, 132, 17381-17383; J. Mater. Chem., 2010, 20, 475; Adv. Mater. 2010, 22, 4355; Mol. Cryst. Liq. Cryst. 2010, 519, 266, and J. Mater. Chem., 2011, 21, 17345 they all disclose the use of other types of fullerene bis-adducts as acceptors for the construction of improved poly(3-hexylthiophene) (P3HT)-based solar cells.

Adv. Funct. Mater., 2005, 15, 1979-1987; Org. Lett., 2007, 9, 551-554, and J. Mater. Chem., 2010, 21, 1382 first described the use of methoxyphenyl or alkoxyphenyl substituents to decrease the electron affinity of cyclopropane-type fullerene derivatives and to increase the voltage of organic solar cells.

WO 2011/160021 A2 demonstrates the use of cyclopropane-type fullerene derivatives comprising tetraalkoxyphenyl substituents to increase the voltage and energy conversion efficiency of organic solar cells.

JP 2012 020949 A2 discloses the use of cyclopropane-type fullerene derivatives with reduced electron affinity.

Some other fullerene derivatives with reduced electron affinity are known from Photovoltaics (2010), 89; Physical Chemistry Chemical Physics (2010), 12(18), 4656; Physica Status Solidi RRL: Rapid Research Letters (2008), 2(6), 260; Am. Chem. Soc., 2001, 123, 6715; Thin Solid Films 2004, 451-452, 43; J. Org. Chem. 2006, 71, 2995; J. Am. Chem. Soc. 2008, 130, 6447; J. Phys. Chem. C, 2009, 113, 21970; J. Fluor. Chem. 2009, 130, 361 and US 20070202413.

The use of pyrrolidine-type fullerene derivatives with benzocyclobutene-containing substituents in photovoltaic cells is known from EP 2 460 725 A1. The use of fulleropyrrolidines with two substituents at positions 1' and 2' in photovoltaic cells is known from EP 2 495 246 A1 and WO 2009/035024 A1.

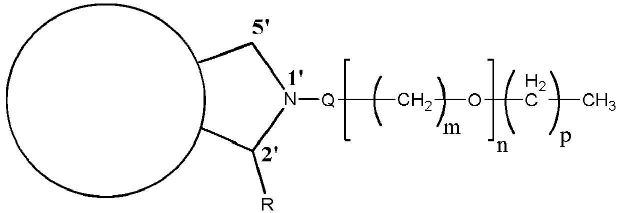

The use of another group of 1',2'-disubstituted fullerene derivatives in photovoltaic cells is known from WO 2009/035017 A1.

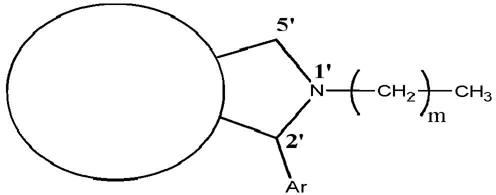

Use of still another group of 1',2'-disubstituted fullerene derivatives in organic photovoltaic cells is known from US2011/0193072 A1.

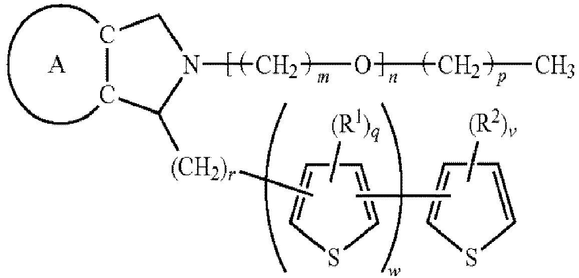

Another group of 1',2'-disubstituted fullerene derivatives is disclosed in US 2011/0193073 A1.

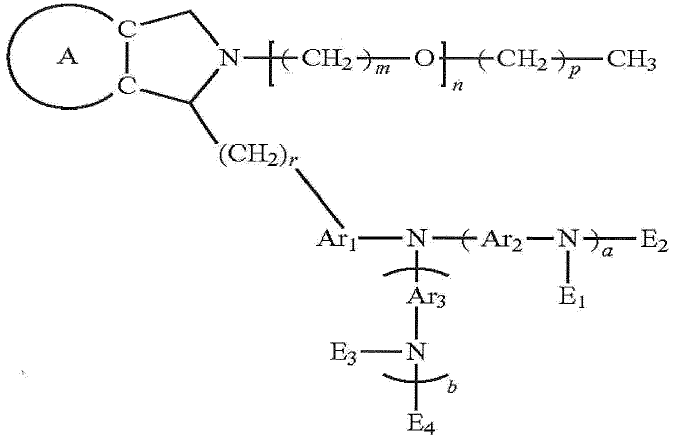

A family of bisfunctionalized fullerene derivatives, where one function is represented by a 1',2'-disubstituted pyrrolidine ring, was disclosed in EP 2 457 898 A1.

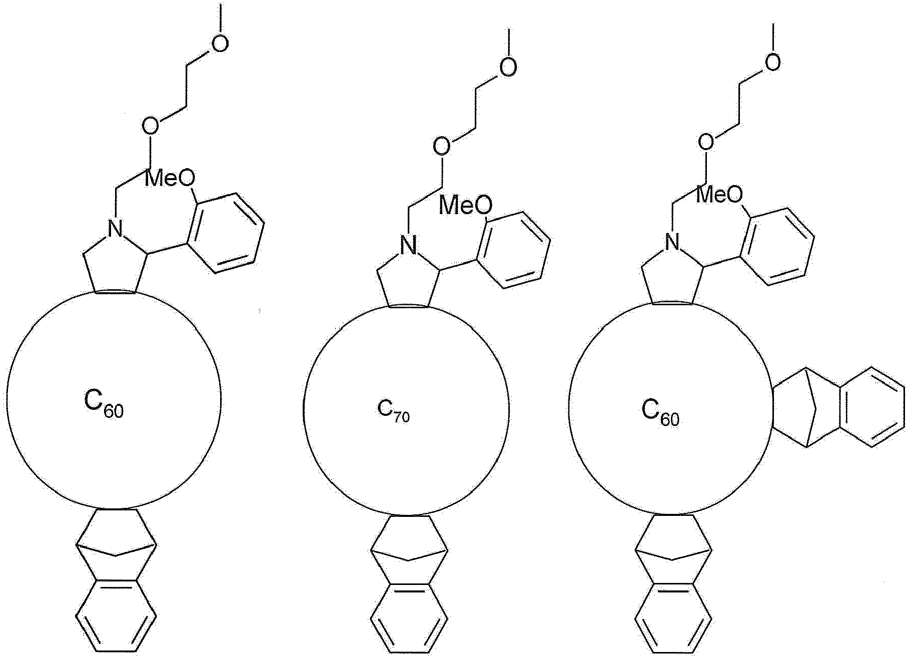

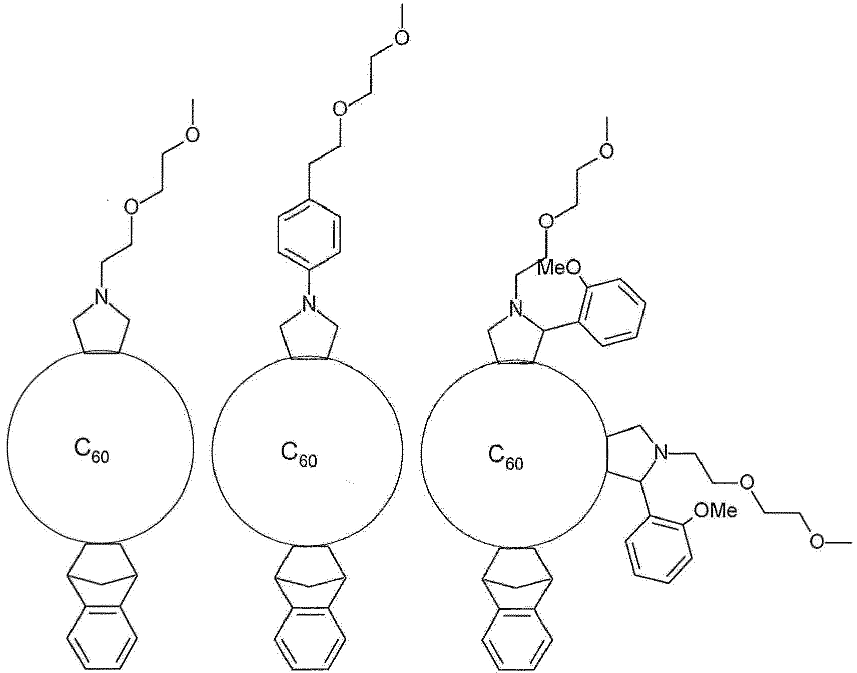

The most successful example demonstrating the use of 1,2'-disubstituted fullerene derivatives, where one function is represented by a pyrrolidine ring in photovoltaic cells, was disclosed in J. Mater. Chem. 2010, 20, 9226. Compound 2PyF-1 yielded photovoltage of 660 mV in combination with P3HT (Poly(3-hexylthiophene-2,5-diyl; [CAS No. 156074-98-5]) and a solar cell power conversion efficiency of 3.44%.

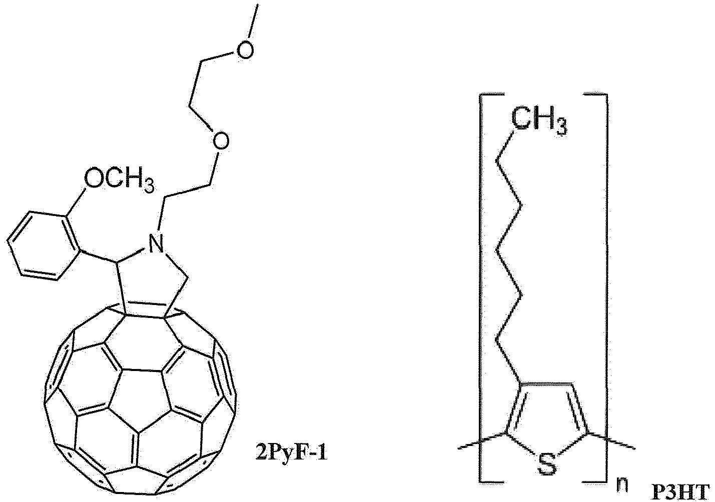

Poly(3-hexylthiophene-2,5-diyl (P3HT) is a polymer from the group of polythiophenes and an organic p-type semiconductor having a molecular weight MW in the range of from 40.000 to 70.000, a molecular weight distribution in the range of from 1,5 to 2,5, a stereoregularity of min. 97% and can be obtained from Sigma Aldrich Inc, St. Louis, USA.

As it is illustrated by the prior art references listed above, the use of 1',2'-disubstituted fulleropyrrolidines, especially ones having a single alkoxyphenyl group, for increasing the voltage in organic solar cells is already known from the prior art. However, it was not possible to achieve significant increase in the energy conversion efficiency of organic solar cells by using such 1',2'-disubstituted fulleropyrrolidines. Typically, rather small increase in the open circuit voltage was counterbalanced by decreased short circuit current and fill factor.

For example, solar cells with fullerene derivatives and P3HT according to US2011/0193072 A1 demonstrated power conversion efficiencies of just 2.3 - 2.5%. Solar cells based on fullerene derivatives with P3HT according to US 2011/0193073 A1 demonstrated a power conversion efficiency of 2.1%. The solar cells based on fullerene derivatives and P3HT according to WO 2009/035024 A1 demonstrated a power conversion efficiency of 2.6 - 2.7%. However, it is well-known that the conventional fullerenes derivative [60]PCBM (Phenyl-C61-butyric acid methyl ester [CAS No. 161196-25-4]) gives routinely power conversion efficiencies above 3.0% in combination with P3HT (see special review in *Adv. Mater.* 2011, *23*, 3597-3602). [60]PCBM, is an effective solution processable n-type organic semiconductor that can be obtained from Sigma Aldrich, St. Louis, USA.

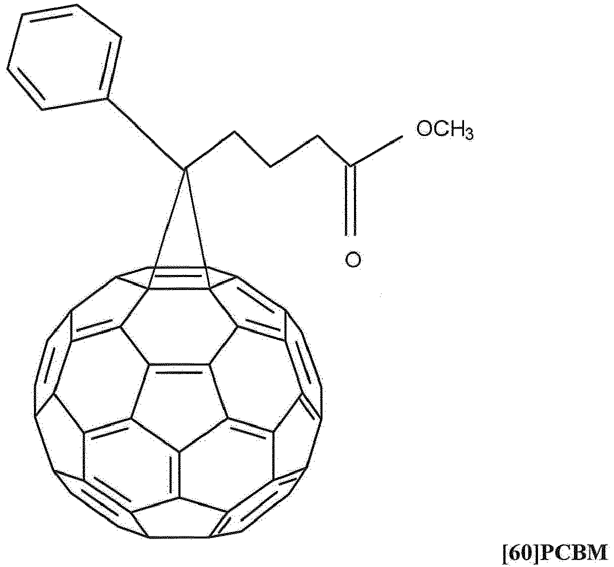

As a result, only 2PyF-1 exhibited a power conversion efficiency comparable to the standard material [60]PCBM. However, even the best combination of prior art fullerene derivatives with P3HT gives just a moderate increase in the open circuit voltage equal to 40-80 mV.

Poor solar cell performance of the 1',2-disubstituted fulleropyrrolidines reported by now in the prior art appears to be related to their unbalanced electronic and physical properties. With respect to the above described disadvantages of 1',2'-disubstituted fulleropyrrolidines with reduced electron affinity, it therefore was an object of the present invention to provide alternative appropriately designed fulleropyrrolidines with
1) considerable improvement in the open circuit voltage of organic solar cells compared to the reference systems where [60]PCBM is used as electron acceptor (e.g. V_{OC} should be above 700 mV in solar cells with P3HT);
2) considerable improvement in the energy conversion efficiency of organic solar cells in comparison to the reference systems where [60]PCBM is used as electron acceptor;
3) and having appropriate physical properties, e.g. sufficient solubility in organic solvents required for solution processing, sufficient thermal stability and photostability to enable long lifetimes of organic solar cells and etc.

The object is achieved by fulleropyrrolidines of formula (1) 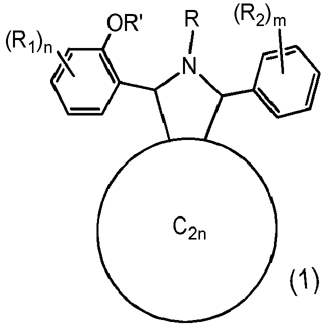 wherein
C₂ₙ is a carbon cage of C₆₀ or C₇₀ fullerene wherein,
R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group,
R₁ and R₂ are the same or independent from each other and represent a substitutent selected from the group of a hydrogen atom, an optionally substituted C₁-C₂₀ alkyl group, a C₁-C₂₀ haloalkyl group, preferably a C₁-C₂₀ fluoroalkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group,
while n and m represent the number of substituents R₁ and R₂ in the corresponding phenyl rings attached to the pyrrolidine unit and are independent from each other an integer from 1 to 3.

As illustrated by the examples of the present invention, the presence of three substituents in the pyrrolidine ring, in particular, aryl substituents in the positions 2' and 5', surprisingly improves the electronic properties of the fullerene core. At the same time, a solubilizing alkyl group or a modified alkyl group attached to the nitrogen atom in position 1' provides optimal solubility and miscibility with conjugated polymers.

For clarification, it should be noted that the scope of the invention encompasses all of process steps, parameters and illustrations set forth above and below, either in general or within areas of preference or preferred embodiments, with one another, i.e., also any combinations between the particular areas and areas of preference. Additionally "[60]" represents a C₆₀ fullerene and "[70]" represents a C₇₀ fullerene and C₂ₙ represents either a C₆₀ fullerene or a C₇₀ fullerene system. Both types of fullerene systems are represented in the present specification by the circled C₂ₙ carbon cage.

In the context of the present invention the prefix "poly" is understood to mean that more than one identical or different repeating units are present, e.g. in the respective compound.

In the light of the foregoing, the present teachings provide fulleropyrrolidines that can be used as organic semiconductor materials. Also provided are associated devices and related methods for the preparation and use of the fulleropyrrolidines according to formula (1). The fulleropyrrolidines according to formula (1) can exhibit properties such as optimized optical absorption, good charge transport characteristics and chemical stability in ambient conditions, low-temperature processability, large solubility in common solvents, and processing versatility (e.g. via various solution processes). As a result, optoelectronic devices, preferably organic solar cells, that incorporate one or more of the fulleropyrrolidines according to formula (1) as a photoactive layer can exhibit high performance in ambient conditions, for example, demonstrating one or more of low band-gap, high fill factor, high open circuit voltage, and high power conversion efficiency, and preferably all of these criteria.

The present invention also provides at least one method for preparing such fulleropyrrolidines of formula (1) and semiconductor materials, as well as various compositions, composites, and devices that incorporate at least one of the fulleropyrrolidines of formula (1) and semiconductor materials disclosed herein.

The foregoing as well as other features and advantages of the present teachings will be more fully understood from the following figures, description, examples, and claims.

It should be understood that the drawings in this specification are for illustration purposes only. The drawings are not necessarily to scale, with emphasis generally being placed upon illustrating the principles of the present teachings. The drawings are not intended to limit the scope of the present teachings in any way.

Throughout the application, where compositions are described as having, including, or comprising specific components, or where processes are described as having, including, or comprising specific process steps, it is contemplated that compositions of the present teachings also consist essentially of, or consist of, the recited components, and that the processes of the present teachings also consist of, the recited process steps.

In the application, where an element or component is said to be included in and/or selected from a list of recited elements or components, it should be understood that the element or component can be any one of the recited elements or components, or the element or component can be selected from a group consisting of two or more of the recited elements or components. Further, it should be understood that elements and/or features of a composition, an apparatus, or a method described herein can be combined in a variety of ways without departing from the spirit and scope of the present teachings, whether explicit or implicit herein.

The use of the terms "include", "includes", "including", "have", "has", or "having" should be generally understood as open-ended and non-limiting unless specifically stated otherwise.

The use of the singular herein includes the plural (and vice versa) unless specifically stated otherwise. In addition, where the use of the term "about" is before a quantitative value, the present teachings also include the specific quantitative value itself, unless specifically stated otherwise. As used herein, the term "about" refers to ± 10% variation from the nominal value unless otherwise indicated or inferred.

It should be understood that the order of steps or order for performing certain actions is immaterial so long as the present teachings remain operable. Moreover, two or more steps or actions may be conducted simultaneously.

As used herein, a "p-type semiconductor material" or a "donor" material refers to a semiconductor material, for example, an organic semiconductor material, having holes as the majority current or charge carriers. In some embodiments, when a p-type semiconductor material is deposited on a substrate, it can provide a hole mobility in excess of about 10⁻⁵ cm²/Vs. In the case of field-effect devices, a p-type semiconductor also can exhibit a current on/off ratio of greater than about 10.

As used herein, an "n-type semiconductor material" or an "acceptor" material refers to a semiconductor material, for example, an organic semiconductor material, having electrons as the majority current or charge carriers. In some embodiments, when an n-type semiconductor material is deposited on a substrate, it can provide an electron mobility in excess of about 10⁻⁵ cm²/Vs. In the case of field-effect devices, an n-type semiconductor also can exhibit a current on/off ratio of greater than about 10.

As used herein, "mobility" refers to a measure of the velocity with which charge carriers, for example, holes (or units of positive charge) in the case of a p-type semiconductor material and electrons (or units of negative charge) in the case of an n-type semiconductor material, move through the material under the influence of an electric field. This parameter, which depends on the device architecture, can be measured using a field-effect device or space-charge limited current measurements or some other techniques like transient photocurrent measurenets, CELIV (current extracted by linearly increased voltage) measurements.

As used herein, the power conversion efficiency (PCE) of a solar cell is the percentage of power converted from absorbed light to electrical energy. The PCE of a solar cell can be calculated by dividing the maximum power point (Pₘ) by the input light irradiance (E, in W/m²) under standard test conditions (STC) and the surface area of the solar cell (A_{c}, in m²). STC typically refers to a temperature of 25°C and an irradiance of 1000 W/m² with an air mass 1.5 (AM 1.5) spectrum.

As used herein, a component (such as a thin film layer) can be considered "photoactive" if it contains one or more compounds that can absorb photons to produce excitons for the generation of a photocurrent.

As used herein, a "polymeric compound" or "polymer" refers to a molecule including a plurality of one or more repeating units connected by covalent chemical bonds. A polymeric compound can be represented by the general formula:

*(̵-M)̵*

wherein M is the repeating unit or monomer. The polymeric compound can have only one type of repeating unit as well as two or more types of different repeating units. When a polymeric compound has only one type of repeating unit, it can be referred to as a homopolymer. When a polymeric compound has two or more types of different repeating units, the term "copolymer" or "copolymeric compound can be used instead. For example, a copolymeric compound can include repeating units

*(̵M^{a})̵* and

*(̵M^{b})̵*

where M^{a} and M^{b} represent two different repeating units. Unless specified otherwise, the assemble of the repeating units in the copolymer can be head-to-tail, head-to-head, or tail-to-tail. In addition, unless specified otherwise, the copolymer can be a random copolymer, an alternating copolymer, or a block copolymer. For example, the general formula:

*(̵M^{a}ₓ-M^{b}_{y})̵*

can be used to represent a copolymer of M^{a} and M^{b} having x mole fraction of M^{a} and or mole fraction of M^{b} in the copolymer, where the manner in which comonomers M^{a} and M^{b} is repeated can be alternating, random, regiorandom, regioregular, or in blocks. In addition to its composition, a polymeric compound can be further characterized by its degree of polymerization (n) and molar mass (e.g., number average molecular weight (Mₙ) and/or weight average molecular weight (M_{w}) depending on the measuring technique(s)).

As used herein, "halo" or "halogen" refers to fluoro, chloro, bromo and iodo, preferably to fluoro. As used herein, "oxo" refers to a double-bonded oxygen (i.e., =O).

As used herein, "alkyl" refers to a straight-chain or branched saturated hydrocarbon group. Preferred alkyl groups include methyl (Me), ethyl (Et), propyl (e.g., n-propyl and iso-propyl), butyl (e.g., n-butyl, iso-butyl, sec-butyl, tert-butyl), pentyl groups (e.g., n-pentyl, iso-pentyl, neo-pentyl), hexyl groups, and the like. In various embodiments, an alkyl group can have 1 to 40 carbon atoms (i.e., C₁₄₀ alkyl group), for example, 1-20 carbon atoms (i.e., C₁₋₂₀ alkyl group). In some embodiments, an alkyl group can have 1 to 6 carbon atoms, and can be referred to as a "lower alkyl group." Examples of lower alkyl groups include methyl, ethyl, propyl (e.g., n-propyl and iso-propyl), and butyl groups (e.g., n-butyl, iso-butyl, secbutyl, tert-butyl). In some embodiments, alkyl groups can be substituted as described herein. An alkyl group is generally not substituted with another alkyl group, an alkenyl group, or an alkynyl group.

As used herein, "haloalkyl" refers to an alkyl group having one or more halogen substituents. At various embodiments, a haloalkyl group can have 1 to 40 carbon atoms (i.e., C₁₋₄₀haloalkyl group), for example, 1 to 20 carbon atoms (i.e., C₁₋₂₀ haloalkyl group). Preferred haloalkyl groups include CF₃, C₂F₅, CHF₂, CH₂F, CCl₃, CHCl₂, CH₂Cl, C₂Cl₅, and the like. Perhaloalkyl groups, i.e., alkyl groups where all of the hydrogen atoms are replaced with halogen atoms (e.g., CF₃ and C₂F₅), are included within the definition of "haloalkyl." For example, a C₁₋₄₀haloalkyl group can have the formula -CₛH₂ₛ₊₁₋ₜ X⁰ₜ, where X⁰, at each occurrence, is F, Cl, Br or I, "s" is an integer in the range of 1 to 40, and "t" is an integer in the range of 1 to 81, provided that t is less than or equal to 2s+1. Haloalkyl groups that are not perhaloalkyl groups can be substituted as described herein.

As used herein, "alkoxy" refers to an -O-alkyl group. Preferred alkoxy groups include, but are not limited to, methoxy, ethoxy, propoxy (e.g., n-propoxy and isopropoxy), t-butoxy, pentoxyl, hexoxyl groups, and the like. The alkyl group in the -O-alkyl group can be substituted as described herein.

As used herein, "alkylthio" refers to an -S-alkyl group. Preferred alkylthio groups include, but are not limited to, methylthio, ethylthio, propylthio (e.g., n-propylthio and isopropylthio), t-butylthio, pentylthio, hexylthio groups, and the like. The alkyl group in the -S-alkyl group can be substituted as described herein.

As used herein, "alkenyl" refers to a straight-chain or branched alkyl group having one or more carbon-carbon double bonds. Preferred alkenyl groups include ethenyl, propenyl, butenyl, pentenyl, hexenyl, butadienyl, pentadienyl, hexadienyl groups, and the like. The one or more carbon-carbon double bonds can be internal (such as in 2-butene) or terminal (such as in 1-butene). In various embodiments, an alkenyl group can have 2 to 40 carbon atoms (i.e., C₂₋₄₀ alkenyl group), for example, 2 to 20 carbon atoms (i.e., C₂₋₂₀ alkenyl group). In some embodiments, alkenyl groups can be substituted as described herein. An alkenyl group is generally not substituted with another alkenyl group, an alkyl group, or an alkynyl group.

As used herein, "alkynyl" refers to a straight-chain or branched alkyl group having one or more triple carbon-carbon bonds. Preferred alkynyl groups include ethynyl, propynyl, butynyl, pentynyl, hexynyl, and the like. The one or more triple carbon-carbon bonds can be internal (such as in 2-butyne) or terminal (such as in 1-butyne). In various embodiments, an alkynyl group can have 2 to 40 carbon atoms (i.e., C₂₋₄₀ alkynyl group), for example, 2 to 20 carbon atoms (i.e., C₂₋₂₀ alkynyl group). In some embodiments, alkynyl groups can be substituted as described herein. An alkynyl group is generally not substituted with another alkynyl group, an alkyl group, or an alkenyl group.

As used herein, a "cyclic moiety" can include one or more (e.g., 1-6) carbocyclic or heterocyclic rings. The cyclic moiety can be a cycloalkyl group, a heterocycloalkyl group, an aryl group, or a heteroaryl group (i.e., can include only saturated bonds, or can include one or more unsaturated bonds regardless of aromaticity), each including, for example, 3-24 ring atoms and optionally can be substituted as described herein. In embodiments where the cyclic moiety is a "monocyclic moiety," the "monocyclic moiety" can include a 3-14 membered aromatic or non-aromatic, carbocyclic or heterocyclic ring. A monocyclic moiety can include, for example, a phenyl group or a 5- or 6-membered heteroaryl group, each of which optionally can be substituted as described herein. In embodiments where the cyclic moiety is a "polycyclic moiety," the "polycyclic moiety" can include two or more rings fused to each other (i.e., sharing a common bond) and/or connected to each other via a spiro atom, or one or more bridged atoms. A polycyclic moiety can include an 8-24 membered aromatic or non-aromatic, carbocyclic or heterocyclic ring, such as a C₈₋₂₄ aryl group or an 8-24 membered heteroaryl group, each of which optionally can be substituted as described herein.

As used herein, "cycloalkyl" refers to a non-aromatic carbocyclic group including cyclized alkyl, alkenyl, and alkynyl groups. In various embodiments, a cycloalkyl group can have 3 to 24 carbon atoms, for example, 3 to 20 carbon atoms (e.g., C₃₋₁₄ cycloalkyl group). A cycloalkyl group can be monocyclic (e.g., cyclohexyl) or polycyclic (e.g., containing fused, bridged, and/or spiro ring systems), where the carbon atoms are located inside or outside of the ring system. Any suitable ring position of the cycloalkyl group can be covalently linked to the defined chemical structure. Examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcaryl, adamantyl, and spiro[4.5]decanyl groups, as well as their homologs, isomers, and the like. In some embodiments, cycloalkyl groups can be substituted as described herein.

As used herein, "aryl" or Ar refers to an aromatic monocyclic hydrocarbon ring system or a polycyclic ring system in which two or more aromatic hydrocarbon rings are fused (i.e., having a bond in common with) together or at least one aromatic monocyclic hydrocarbon ring is fused to one or more cycloalkyl and/or cycloheteroalkyl rings. An aryl group prefereably has 6 to 24 carbon atoms in its ring system (e.g., C₆₋₂₀ aryl group), which can include multiple fused rings. In some embodiments, a polycyclic aryl group can have 8 to 24 carbon atoms. Any suitable ring position of the aryl group can be covalently linked to the defined chemical structure. Preferred aryl groups having only aromatic carbocyclic ring(s) include phenyl, 1-naphthyl (bicyclic), 2-naphthyl (bicyclic), anthracenyl (tricyclic), phenanthrenyl (tricyclic), pentacenyl (pentacyclic), and the like. Preferred polycyclic ring systems in which at least one aromatic carbocyclic ring is fused to one or more cycloalkyl and/or cycloheteroalkyl rings include, among others, benzo derivatives of cyclopentane (i.e., an indanyl group, which is a 5,6-bicyclic cycloalkyl/aromatic ring system), cyclohexane (i.e., a tetrahydronaphthyl group, which is a 6,6-bicyclic cycloalkyl/aromatic ring system), imidazoline (i.e., a benzimidazolinyl group, which is a 5,6-bicyclic cycloheteroalkyl/aromatic ring system), and pyran (i.e., a chromenyl group, which is a 6,6-bicyclic cycloheteroalkyl/aromatic ring system). Other preferred aryl groups include benzodioxanyl, benzodioxolyl, chromanyl, indolinyl groups, and the like. In some embodiments, aryl groups can be substituted as described herein. In some embodiments, an aryl group can have one or more halogen substituents, and can be referred to as a "haloaryl" group. Perhaloaryl groups, i.e., aryl groups where all of the hydrogen atoms are replaced with halogen atoms (e.g., -C₆F₅), are included within the definition of "haloaryl." In certain embodiments, an aryl group is substituted with another aryl group and can be referred to as a biaryl group. Each of the aryl groups in the biaryl group can be substituted as disclosed herein.

In contrast to monovalent groups compounds of the present teachings can include a "divalent group" defined herein as a linking group capable of forming a covalent bond with two other moieties. For example, compounds of the present teachings can include a divalent C₁₋₂₀ alkyl group (e.g., a methylene group), a divalent C₂₋₂₀ alkenyl group (e.g., a vinylyl group), a divalent C₂₋₂₀ alkynyl group (e.g., an ethynylyl group), a divalent C₆₋₁₄ aryl group (e.g., a phenylyl group), a divalent 3-14 membered cycloheteroalkyl group (e.g., a pyrrolidylyl), and/or a divalent 5-14 membered heteroaryl group (e.g., a thienylyl group). Generally, a chemical group (e.g. -Ar-) is understood to be divalent by the inclusion of the two bonds before and after the group.

At various places in the present specification, substituents are disclosed in groups or in ranges. It is specifically intended that the description includes each and every individual subcombination of the members of such groups and ranges. For example, the term "C₁₋₆ alkyl" is specifically intended to individually disclose C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆ alkyl. By way of other examples, an integer in the range of 0 to 40 is specifically intended to individually disclose 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40, and an integer in the range of 1 to 20 is specifically intended to individually disclose 1, 2, 3, 4, 5, 6, 7, 8, 9, 10, 11, 12, 13, 14, 15, 16, 17, 18, 19, and 20. Additional examples include that the phrase "optionally substituted with 1-5 substituents" is specifically intended to individually disclose a chemical group that can include 0, 1, 2, 3, 4, 5, 0-5, 0-4, 0-3, 0-2, 0-1, 1-5, 1-4, 1-3, 1-2, 2-5, 2-4, 2-3, 3-5, 3-4, and 4-5 substituents.

Fulleropyrrolidines according to formula (1) can contain an asymmetric atom (also referred as a chiral center) and some of the fulleropyrrolidines can contain two or more asymmetric atoms or centers, which can thus give rise to optical isomers (enantiomers) and geometric isomers (diastereomers). The present teachings include such optical and geometric isomers, including their respective resolved enantiomerically or diastereomerically pure isomers (e.g., (+) or (-) stereoisomer) and their racemic mixtures, as well as other mixtures of the enantiomers and diastereomers. In some embodiments, optical isomers can be obtained in enantiomerically enriched or pure form by standard procedures known to those skilled in the art, which include, for example, chiral separation, diastereomeric salt formation, kinetic resolution, and asymmetric synthesis. The present teachings also encompass cis- and trans-isomers of fulleropyrrolidines containing alkenyl moieties (e.g., alkenes, azo, and imines). It also should be understood that the fulleropyrrolidines of formula (1) encompass all possible regioisomers in pure form and mixtures thereof. In some embodiments, the preparation of the present fulleropyrrolidines can include separating such isomers using standard separation procedures known to those skilled in the art, for example, by using one or more of column chromatography, thin-layer chromatography, simulated moving-bed chromatography, and high-performance liquid chromatography. However, mixtures of regioisomers can be used similarly to the uses of each individual regioisomer of the present teachings as described herein and/or known by a skilled artisan. It is specifically contemplated that the depiction of one regioisomer includes any other regioisomers and any regioisomeric mixtures unless specifically stated otherwise.

Throughout the specification, structures may or may not be presented with chemical names. Where any question arises as to nomenclature, the structure prevails.

In a preferred embodiment the present invention relates to fulleropyrrolidines of formula (1) wherein R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group and R₁ and R₂ are the same or independent from each other and represent an optionally substituted C₁-C₂₀ alkyl group, a C₁-C₂₀ fluorinated alkyl group, a C₁-C₂₀ fluorinated alkylether group, C₁-C₂₀ alkoxy group, a C₁-C₂₀ fluoroalkoxy group, a C₁-C₂₀ alkylthio group or a C₁-C₂₀ fluoroalkylthio group, while n=2 and m=3.

In a preferred embodiment the present invention relates to fulleropyrrolidines of formula (1) wherein R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group and R₁ and R₂ are the same or independent from each other they represent a C₁-C₂₀ alkoxy group, a C₁-C₂₀ fluoroalkoxy group, a C₁-C₂₀ alkylthio group or a C₁-C₂₀ fluoroalkylthio group, while n = 1 or 2 and m = 1, 2 or 3.

In a very preferred embodiment the present invention relates to fulleropyrrolidines of formula (1) wherein R and R` are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group and R₁ and R₂ in formula (1) are the same or independent from each other and represent C₁-C₂₀ alkoxy group or C₁-C₂₀ alkylthio group, while n = 1 or 2 and m = 1, 2 or 3.

In another very preferred embodiment the present invention relates to fulleropyrrolidines of formula (1) wherein R and R` are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group and R₁ and R₂ are the same or independent from each other and represent a C₁-C₂₀ alkoxy group or a C₁-C₂₀ alkylthio group, while n = 2 and m = 3.

In a preferred embodiment the present invention refers to a fulleropyrrolidine of formula (1) wherein R represents a substitutent selected from the group of an optionally substituted C₁-C₂₀ alkyl group or a C₁-C₂₀ alkylether group.

In another preferred embodiment the present invention refers to a fulleropyrrolidine of formula (1) wherein R' represents a substitutent selected from the group of an optionally substituted C₁-C₂₀ alkyl group or a C₁-C₂₀ alkylether group.

In another preferred embodiment the present invention refers to a fulleropyrrolidine of formula (1) wherein R₁ and R₂ are the same or independent from each other and represent a substitutent selected from the group of a hydrogen atom, a C₁-C₂₀ alkoxy group or a C₁-C₂₀ alkylthio group,

In another preferred embodiment the present invention refers to a fulleropyrrolidine of formula (1) wherein R₁ and R₂ are the same or independent from each other and represent a hydrogen atom or a C₁-C₂₀ alkoxy group,

In another preferred embodiment the present invention refers to a fulleropyrrolidine of formula (1) wherein R₁ and R₂ are the same or independent from each other and represent a hydrogen atom or a C₁-C₂₀ alkylthio group.

In a special preferred embodiment the present invention relates to fulleropyrrolidines of formulas (1a), (1b), (1c) or (1d) having two, three or four electron donating substituents at positions 2" and 6" of the phenyl rings attached to the pyrrolidine units, 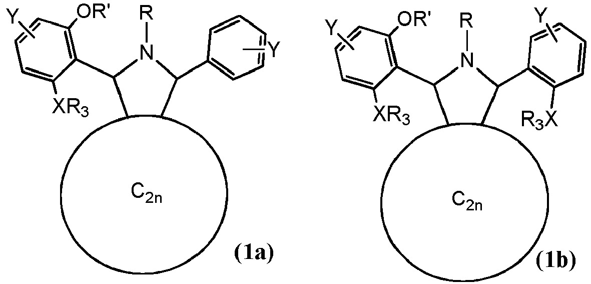 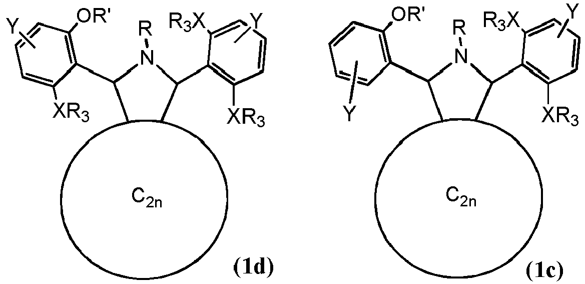
wherein C₂ₙ is a carbon cage of C₆₀ fullerene or a C₇₀ fullerene, preferably a C₆₀ fullerene;
X represents O or S;
R, R' and R₃ are the same or independent from each other and represent a substituent selected from the group of an optionally substituted C₁-C₂₀ alkyl group, a C₁-C₂₀ fluorinated alkyl group or a C₁-C₂₀ alkylether group and Y represents a substitutent selected from the group of a hydrogen atom, an optionally substituted C₁-C₂₀ alkyl chain, a C₁-C₂₀ fluorinated alkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group.

In a very special embodiment the present invention relates to fulleropyrrolidines of formulas (a1-a6), (b1-b6), (c1-c6), (d1-d6), (e1-e6) and (f1-f6) 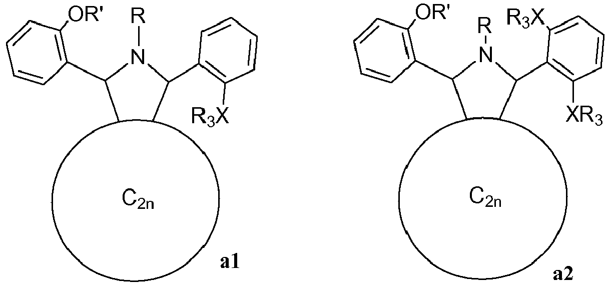 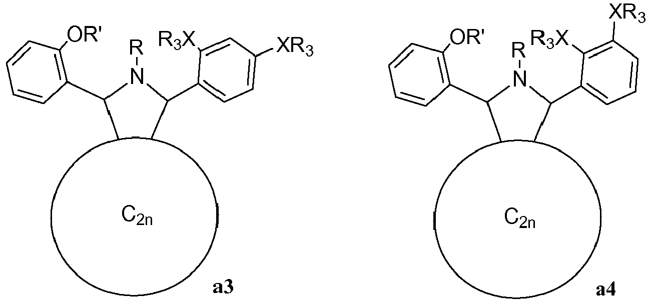 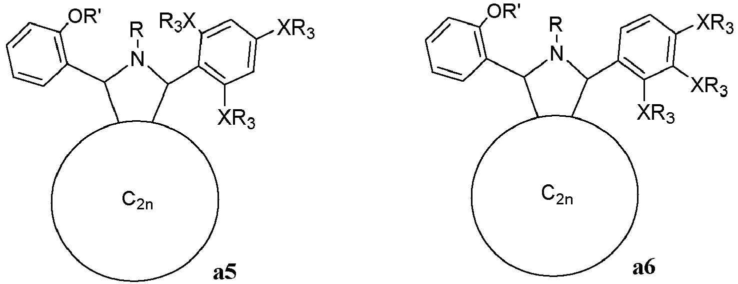 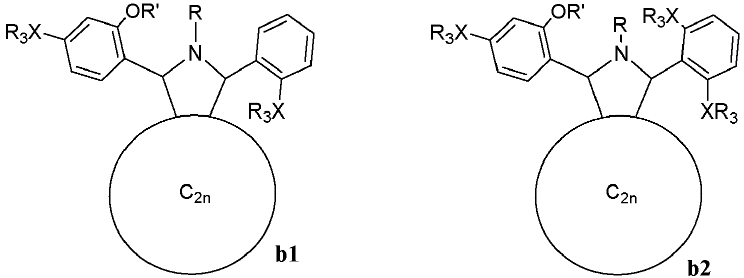 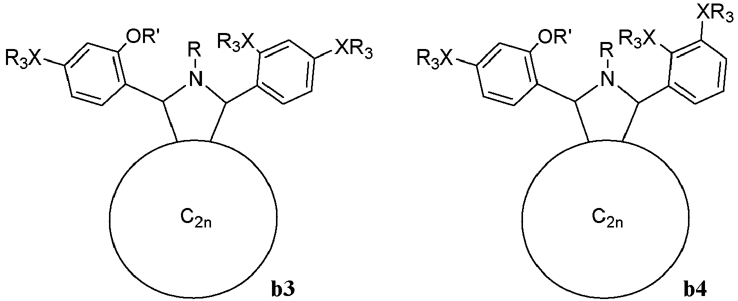 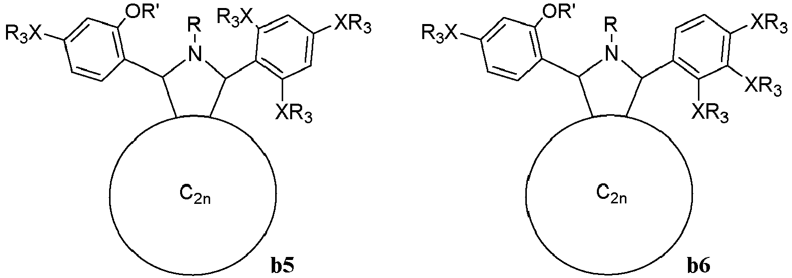 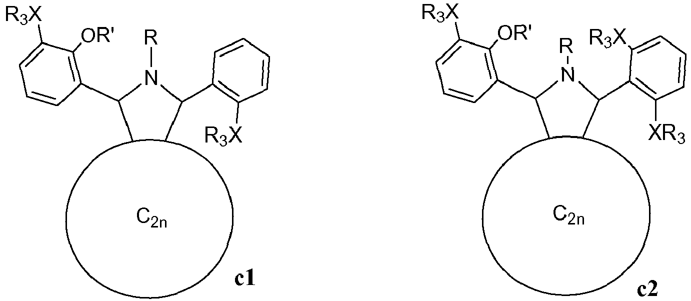 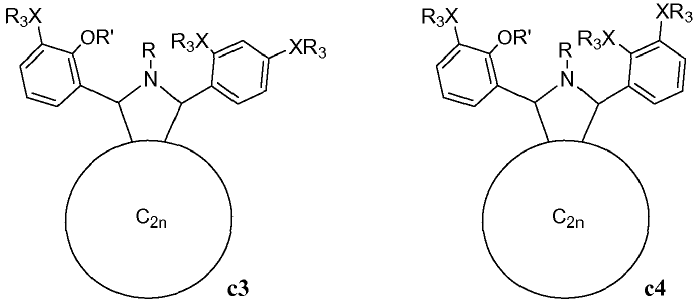 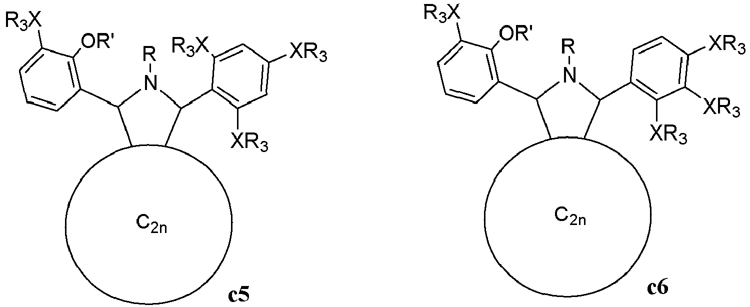 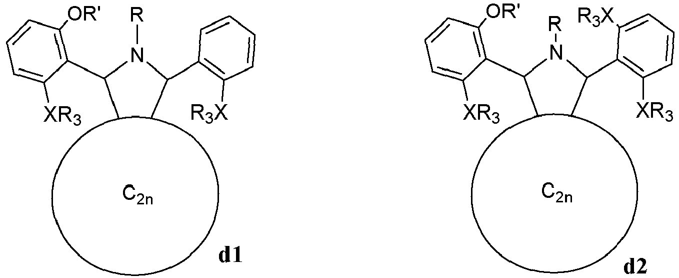 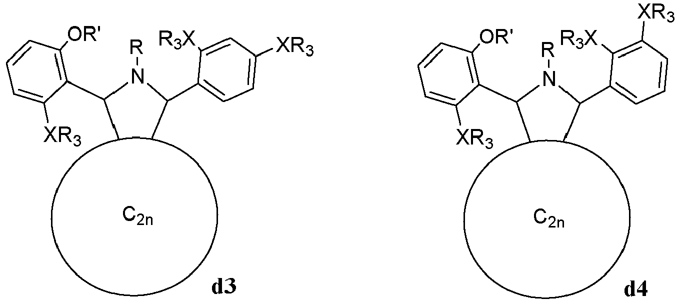 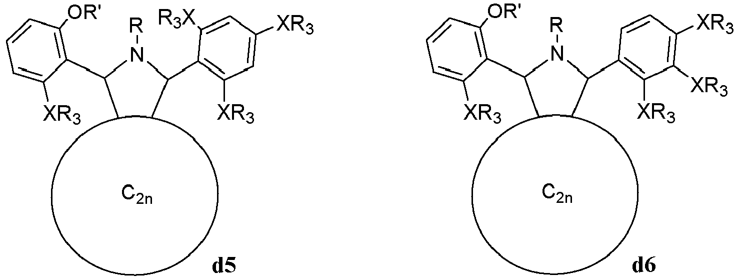 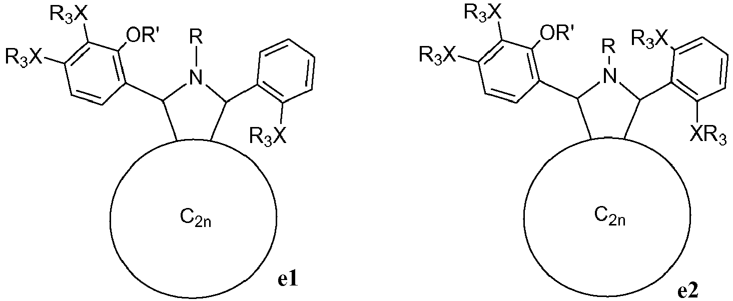 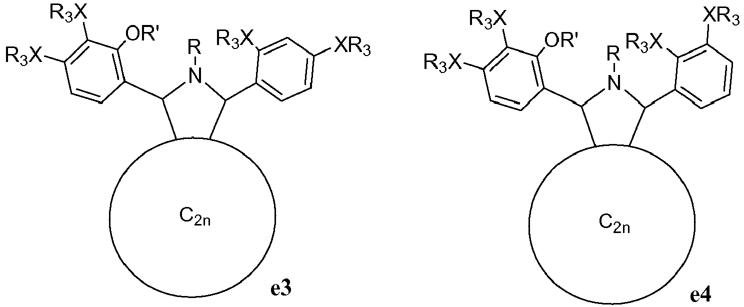 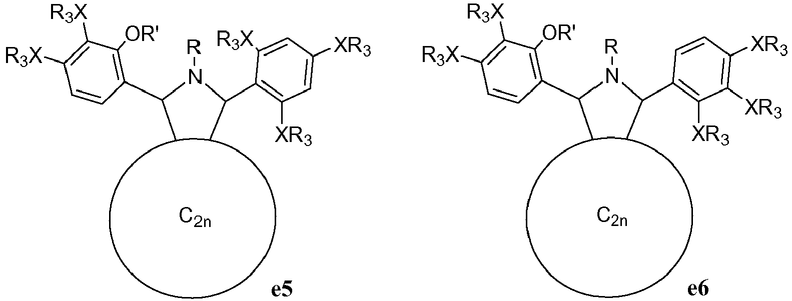 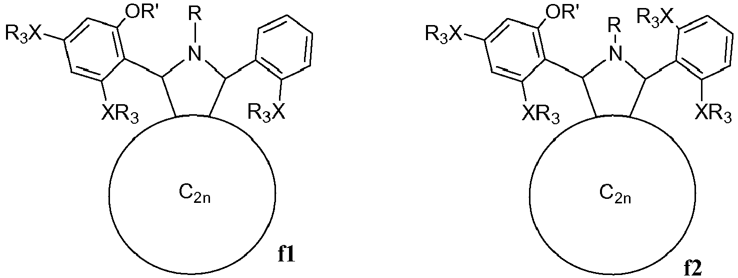 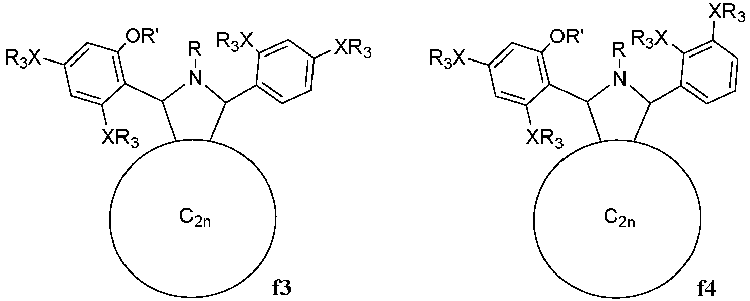 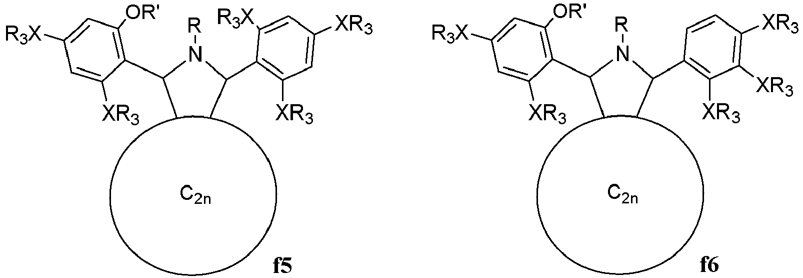 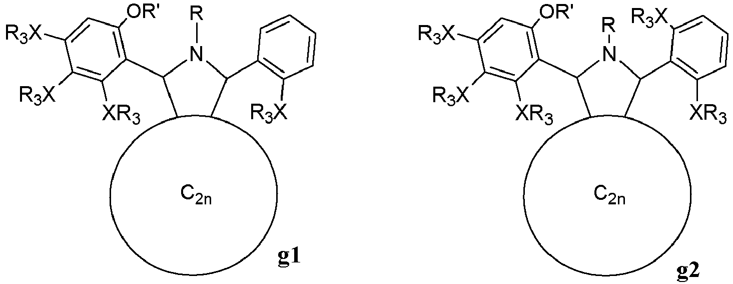 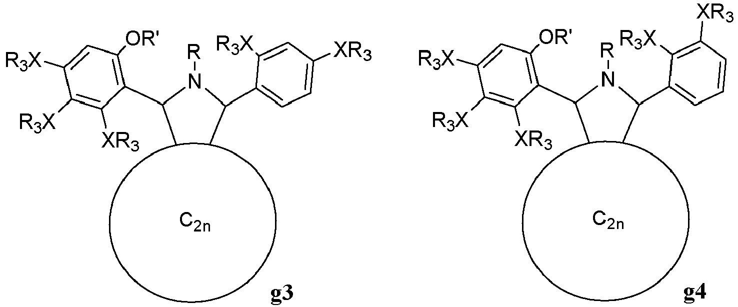 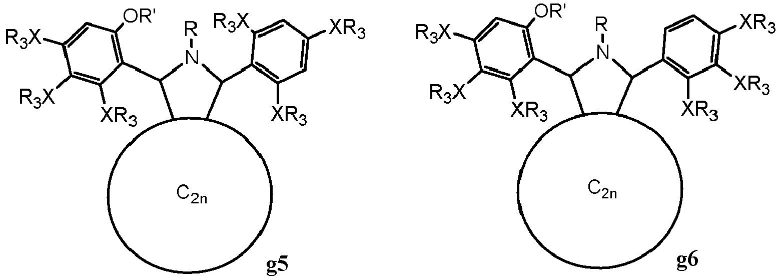
wherein C₂ₙ is a carbon cage of C₆₀ fullerene or C₇₀ fullerene, preferably C₆₀ fullerene;
X represents O or S;
R, R' and R₃ are the same or independent from each other and represent a substituent selected from the group of an optionally substituted C₁-C₂₀ alkyl group, a C₁-C₂₀ fluorinated alkyl group or a C₁-C₂₀ alkylether group.

In a very preferred embodiment of the present invention the fulleropyrrolidines according to the present invention are used in a mixture of at least any two fulleropyrrolidines of general formula (1) taken in any appropriate ratio in combination with 0.0001 to 99.9999% of a third component which might be represented by some functionalized higher fullerene C>70, some solvent, some processing additive but any other functional component improving or not affecting the performance of the fullerene derivatives in the claimed optoelectronic devices, preferably in organic photovoltaic cells.

The fulleropyrrolidines of formula (1) are synthesized using methods known in the prior art. The present invention is therefore directed to a process of manufacturing fulleropyrrolidines of formula (1) according to the scheme 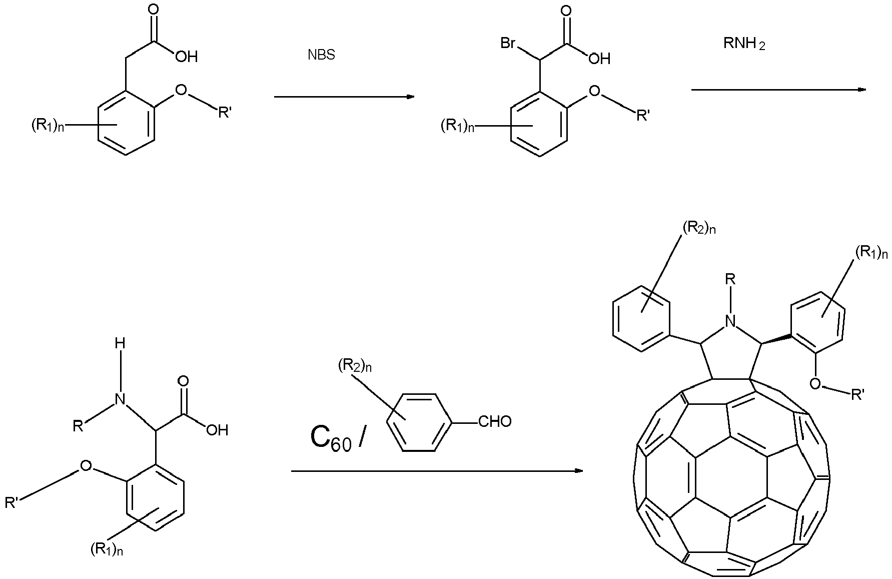 wherein R, R`, R₁, R₂ and n having the same meanings as given above. Characteristic is a [2+3]cycloaddition reaction of azomethine ylides to the fullerene cage named after Prato. Such [2+3]cycloaddition reaction preferably takes place in 1,2-dichlorobenzene under heating at 90-180°C. The Prato reaction in fullerene chemistry describes the functionalization of fullerenes and nanotubes with azomethine ylides in a 1,3-dipolar cycloaddition. The amino acid sarcosine reacts with paraformaldehyde when heated at reflux in toluene to an ylide which reacts with a double bond in a 6,6 ring position in a fullerene in a 1,3-dipolar cycloaddition to yield a N-methylpyrrolidine derivative or pyrrolidinofullerene or pyrrolidino[[3,4:1,2]] [60]fullerene in 82% yield (M. Maggani, G.Scorrano, M.Prato, J. Am. Chem. Soc. 1993, 115 (21), pp 9798-9799).

In a preferred embodiment of the present invention the product mixture obtained by the reaction shown above is processed using column chromatography on silica using toluene-hexane mixtures as eluent to isolate the fulleropyrrolidines of formula (1). Preferably additional preparative HPLC processing using a Buckyprep column might be required to achieve 99+% purity of the fulleropyrrolidines of formula (1). Such Buckyprep column is available from SES Research, Houston, TX 77092 USA.

The present invention therefore refers to a process for the manufacture of fulleropyrrolidines of formula (1) 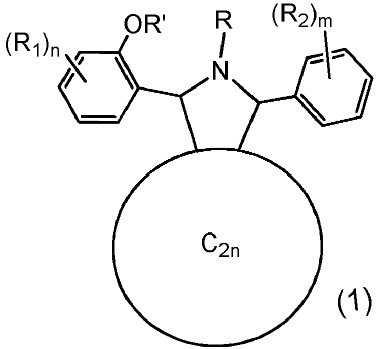 wherein
C₂ₙ is a carbon cage of C₆₀ or C₇₀ fullerene wherein,
R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group,
R₁ and R₂ are the same or independent from each other and represent a substitutent selected from the group of a hydrogen atom, an optionally substituted C₁-C₂₀ alkyl group, a C₁-C₂₀ haloalkyl group, preferably a C₁-C₂₀ fluoroalkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group,
while n and m represent the number of substituents R₁ and R₂ in the corresponding phenyl rings attached to the pyrrolidine unit and are independent from each other an integer from 1 to 3,
characterized in that such process is performed according to the scheme 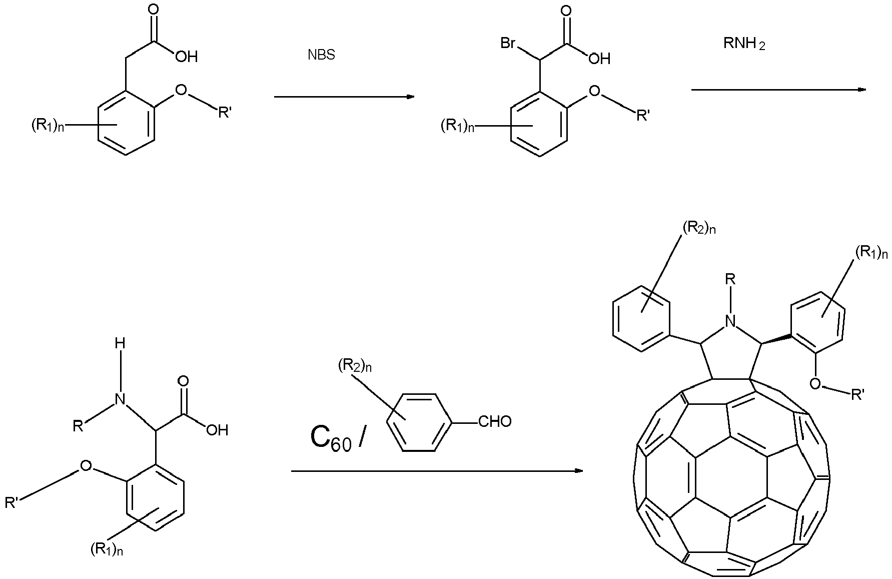 wherein NBS represents 1-Bromo-2,5-pyrrolidinodione [CAS No. 128-08-5], and R, R', R₁, R₂, m and n have the meanings given for formula (I). Preferably the final step of such process is carried out at temperatures in the range of from 120 to 180° C in 1,2 Dichlorobenzene.

A very preferred object of the present invention are fulleropyrrolidines of formula (1e) 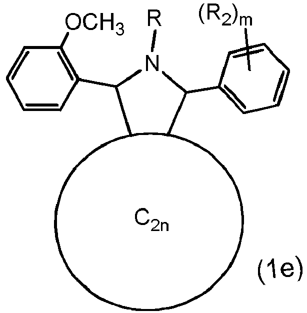
wherein C₂ₙ is a carbon cage of C₆₀ fullerene or C₇₀ fullerene, preferably C₆₀ fullerene;
R represents an optionally substituted C₁-C₂₀ alkyl group;
R₂ represents an otionally substituted C₁-C₂₀ alkoxy group or a C₁-C₂₀ alkylether group,
while m is integer from 1 to 3.

In another very preferred embodiment the present invention is directed to fulleropyrrolidines of formula (1e), 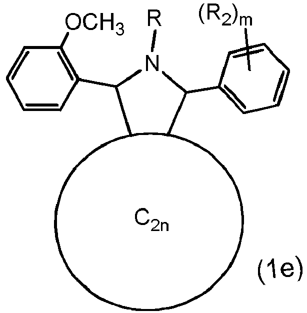
wherein C₂ₙ represents a C₆₀ fullerene system;
R represents an optionally substituted C₁-C₈ alkyl group preferably a C₈ alkyl group;
R₂ represents an optionally substituted C₁-C₅ alkoxy group or a C₁-C₁₀ alkylether group,
while m is integer from 1 to 3.

Particularly preferred are the fulleropyrrolidines of formulas (1f), (1g), (1h). (1i), (1j) and (1k) according to Table 1.

**Table 1**

| No. | fulleropyrrolidines |
|---|---|
| (1f) | 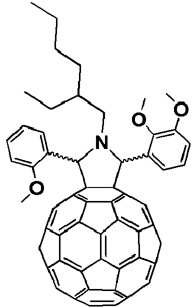 |
| (1g) | 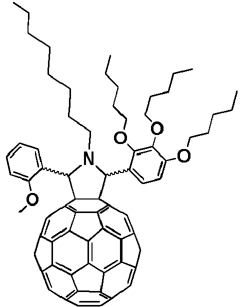 |
| (1h) | 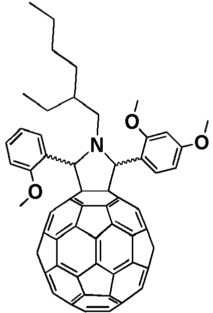 |
| (1i) | 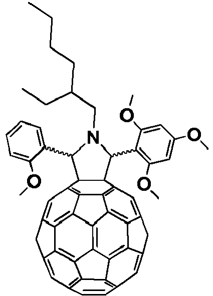 |
| (1j) | 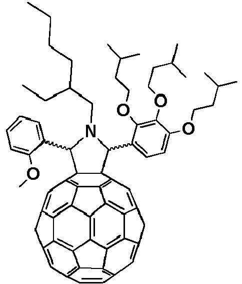 |
| (1k) | 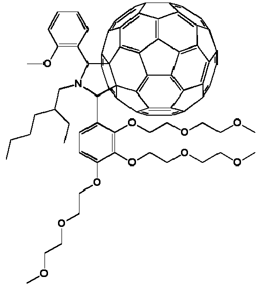 |

Surprisingly the fulleropyrrolidines of formula (1) have unexpectedly lower electron affinity compared to the reference material [60]PCBM. The first electrochemical reduction potential of the fullerene derivative determined as described in J. Org. Chem. 1995, 60, 532 can be considered as a measure of the electronic affinity. [60]PCBM has a reduction potential of -1.135 V vs Fc/Fc⁺. Fullerene derivatives with lower electron affinity should show more negative first reduction potentials (e.g. -1.140 V).

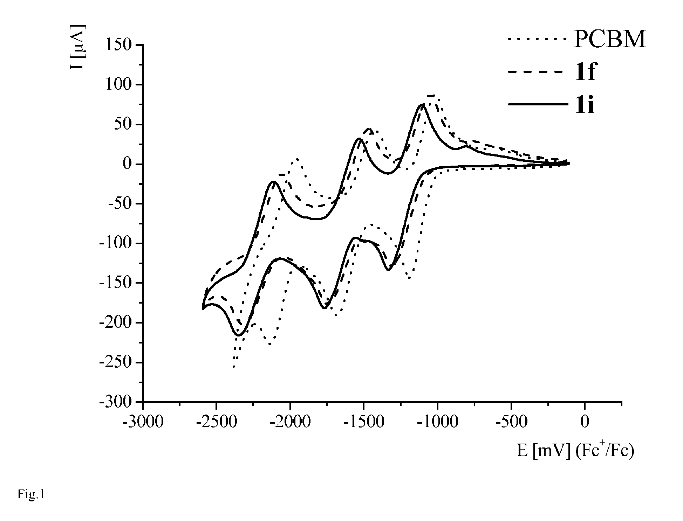

Without limiting the present invention it was found, that the fulleropyrrolidines of formula (1), particularly having 2- 4 alkoxy groups at 2" and 6" positions of the phenyl substituents in the pyrrolidine ring, especially fulleropyrrolidines according to formula (1f) and (1i), have considerably lower reduction potentials compared to [60]PCBM. Fig. 1 demonstrates cyclic voltammograms (CVAs) for fulleropyrrolidines (1f), (1i) and reference [60]PCBM. It shows that the reduction waves of fulleropyrrolidines according to formula (1f) and (1i) are considerably shifted to the negative potentials (cathodic shift) with respect to the corresponding waves of the reference [60]PCBM.

Lower electron affinity of fulleropyrrolidines of formula (1) was observed in solution and in composite films with conjugated polymers (Table 2).

**Table 2 Electrochemical properties and calculated LUMO energy levels of fulleropyrrolidines**

| **Fullerene derivative** | **Solution** | | | **Composite film** | |
|---|---|---|---|---|---|
| | **E¹_{1/2}, V vs. Fc/Fc⁺** | **ΔE, mV*** | **LUMO energy, eV** | **P3HT:Fullerene derivative (1:3)** | |
| | | | | **E¹_{1/2}, V vs. Fc/Fc⁺** | **ΔE, mV*** |
| [60]PCBM | -1,10 | 0 | -4,00 | -1,10 | 0 |
| **1f** | -1,18 | 80 | -3,92 | -1,16 | 60 |
| **1g** | -1,15 | 50 | -3,95 | -1,16 | 60 |
| **1h** | -1,20 | 100 | -3,90 | -1,17 | 70 |
| **1i** | -1,22 | 120 | -3,88 | -1,18 | 80 |
| **1j** | -1,17 | 70 | -3,93 | -1,18 | 80 |
| **1k** | -1,18 | 80 | -3,94 | -1,19 | 90 |

| | | | | | |
|---|---|---|---|---|---|
| *Calcucated as ΔE= -[E¹_{1/2}(1f-k)- E¹_{1/2}[60]PCBM]·1000 mV | | | | | |

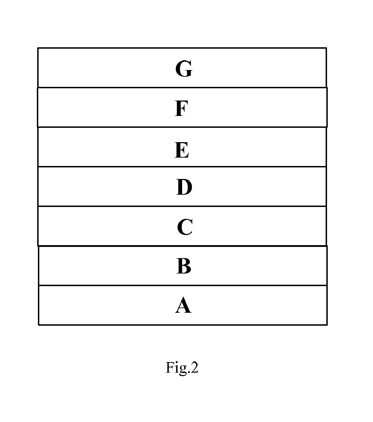

A further object of the present invention is also an organic photovoltaic cell comprising at least one fulleropyrrolidine of formula (1) and having an architecture preferably, as shown in Fig.2. Fig.2 shows a layout of a photovoltaic cell comprising the following components:
a transparent or semitransparent or non-transparent substrate (A);
a bottom electrode (B), which collects the holes from the device;
a hole-selective buffer layer (C), which blocks the electrons and non-dissociated excitations and conducts the holes;
a photoactive layer (D) which functions as generator of free charge carriers under light irradiation and comprises at least one fulleropyrrolidine of the formula (1);
an electron selective buffer layer (E) which blocks the holes and non-dissociated charge carriers and conducts the electrons;
a top electrode (F) which collects the electrons;
a substrate (G) which might be transparent, semitransparent or non-transparent.

In another embodiment of the present invention the components of the photovoltaic cell are arranged in reverse (or "inverted order") as follows:
a transparent or semitransparent or non-transparent substrate (A);
a bottom electrode (B) which collects the electrons;
an electron selective buffer layer (C) which blocks the holes and non-dissociated charge carriers and conducts the electrons;
a photoactive layer (D) which functions as generator of free charge carriers under light irradiation and comprises at least one fulleropyrrolidine of the formula (1);
a hole-selective buffer layer (E), which blocks the electrons and non-dissociated excitations and conducts the holes;
a top electrode (F), which collects the holes from the device;
a substrate (G) which might be transparent, semitransparent or non-transparent.

An embodiment of the present invention is the use of at least one fulleropyrrolidine of formula (1) in a thin film of a photoactive layer, preferably in a thin film of a photovoltaic cell, more preferred in a thin film of an organic photovoltaic cell or of an organic solar cell.

Preferably the fulleropyrrolidines according to formula (1) are used in a composition with at least one electron donor component represented by a fully conjugated or partially conjugated polymer, a low molecular weight compound or inorganic nanoparticle.

Preferred electron donor components are fully conjugated or partially conjugated polymers selected from the group of poly(3-hexylthiophene) P3HT, poly(2,7-(9,9-di(alkyl)-fluorene)-alt-5,5-(4',7'-di-2-thienyl-2',1',3'-benzothiadiazole)) (PFDTBT), poly(2,6-(4,4-bis-(2'-ethylhexyl)-4H-cyclopenta(2,1-b;3,4-6')dithiophene)-alt-4',7'-(2',1',3'-benzothiadiazole) (PCPDTBT), poly(2,6-(4,4-di(n-dodecyl)-4H-cyclopenta(2,1-b;3,4,-6')dithiophene)alt-5,5-(4',7'-di-2-thienyl-2',1',3'- benzothiadiazole)), poly[N-9'-heptadecanyl-2,7-carbazole-alt-5,5-(4',7'-di-2-thienyl-2',1',3'-benzothiadiazole) (PCDTBT).

Preferred low-molecular weight donor materials are zinc or copper, phthalocyanines, thiophene oligomers, organic dyes and other organic compounds characterized by their ability to form stable cationic species under chemical, photo- or electrochemical oxidation.

Preferred inorganic nanoparticles are PbS, PbSe, PdTe, and other types of compounds capable of the electron donation to the fulleropyrrolidines of formula (1) under light irradiation.

In an especial preferred embodiment the present inventions refers to compositions of at least one fulleropyrrolidine of formula (1) with at least one donor component being poly(3-hexylthiophene) P3HT.

As such composition is used in the photoactive layer of a organic solar cell, a preferred embodiment of the present invention is the photoactive layer (D) of an organic solar cell according to Fig. 2 comprising at least one fulleropyrrolidine of the formula (1).

In a preferred embodiment of the present invention the photoactive layer (D) of an organic solar cell according to Fig. 2 comprises at least one fulleropyrrolidine of the formula (1) in combination with at least one electron donor component. Preferably such an electron donor component is selected from (i) a fully conjugated or partially conjugated polymer; (ii) a low molecular weight donor material; (iii) inorganic particles. Preferably a fully conjugated or partiall conjugated polymer (i) is selected from the group of poly(3-hexylthiophene) P3HT, poly(2,7-(9,9-di(alkyl)-fluorene)-alt-5,5-(4',7'-di-2-thienyl-2',1',3'-benzothiadiazole)) (PFDTBT), poly(2,6-(4,4-bis-(2'-ethylhexyl)-4H-cyclopenta(2,1-b;3,4-6')dithiophene)-alt-4',7'-(2',1',3'-benzothiadiazole) (PCPDTBT), poly(2,6-(4,4-di(n-dodecyl)-4H-cyclopenta(2,1-b;3,4,-6')dithiophene)alt-5,5-(4',7'-di-2-thienyl-2',1',3'-benzothiadiazole)) and poly[N-9'-heptadecanyl-2,7-carbazole-alt-5,5-(4',7'-di-2-thienyl-2',1',3'-benzothiadiazole) (PCDTBT). Preferably a low molecular weight donor material (ii) is zinc or copper, phthalocyanines, thiophene oligomers, organic dyes and other organic compounds characterized by their ability to form stable cationic species under chemical, photo- or electrochemical oxidation. Preferred inorganic nanoparticles (iii) are PbS, PbSe, PdTe, and other types of compounds capable of the electron donation to the fulleropyrrolidines of formula (1) under light irradiation.

In a very preferred embodiment of the present invention the photoactive layer (D) of an organic solar cell according to Fig.2 comprises 1-99% of at least one fulleropyrrolidine of formula (1) in a combination with 99 -1% of at least one electron donor component.

In an especially preferred embodiment at least one fulleropyrrolidine of formula (1), preferably at least one compound of the formulas (1a), (1b), (1c), (1d), more preferred at least one compound of formula (1e) is used in combination with poly(3-hexylthiophene).

In another very preferred embodiment of the present invention the photoactive layer (D) of a solar cell according to Fig. 2 comprises a composition of at least one fulleropyrrolidine of formula (1) with at least one donor component in combination with a third component selected from another electron donor compound, another electron acceptor compound, a processing additive, traces of solvents or other impurities improving or not affecting significantly the performance of the entire photovoltaic device.

In another very preferred embodiment of the present invention the photoactive layer (D) of a solar cell according to Fig. 2 comprises 1-99.999% of a composition of at least one fulleropyrrolidine of formula (1) with at least one donor component in combination with 0.001-99% of a third component which can be another electron donor compound, another electron acceptor compound, a processing additive, traces of solvents or other impurities improving or not affecting significantly the performance of the entire photovoltaic device.

In a preferred embodiment of the present invention one substrate, preferably (A) or (G), in a solar cell according to Fig. 2 is semitransparent or transparent. As referred to herein, the transparent material is a material which, at the thickness used in a photovoltaic cell, transmits at least 70% of incident solar light, preferably up to 75%, especially preferred up to 95%. Semitransparent material is a material which, at the thickness used in a photovoltaic cell, transmits from 1 to 70% of the incident solar light. Non-transparent material is a material which, at the thickness used in photovoltaic cell, transmits less than 1% of the incident solar light.

In a very preferred embodiment of the present invention both substrates, preferably (A) and (G) are semitransparent or transparent. Semitransparent or transparent photovoltaic cells are demonstrated for example in the product brochure for arnoldglas VOLTARLUX®, Glaswerke Arnold, Merkendorf, Germany.

In a preferred embodiment of the present invention the substrates (A) and (G) in a solar cell according to Fig. 2 have a thickness above 0.1 µm (e.g. 0.2 µm or 1µm) and below 2000 µm, more preferred in the range from 500 µm to 1000 µm.

In a preferred embodiment of the present invention the substrates (A) and (G) in a solar cell according to Fig. 2 are represented by flexible plastic (organic or hybrid) foils with an oxygen transmission rate (OTR) below 10⁻² cc/ m² d and water transmission rate (WTR) below 10⁻² g/(m² d).

In a preferred embodiment of the present invention one of the substrates (A) or (G) in a solar cell according to Fig. 2 is represented by a thin metal foil.

In a very preferred embodiment of the present invention the flexible plastics to be used as the substrates can be composed of at least one compound of the group polyethylene terephthalates, polyimides, polypropylene, polyethylene, cellulosic polymers, polyethers, polyamides, polyketones, or compositions of at least two of these materials with each other, or compositions of at least one of the aforementioned materials with inorganic components, preferably metal oxides, phosphates, nitrides and other functional materials forming compact continuous films.

In one of the possible embodiments of the present invention external, internal or both surfaces of the substrates (A) and (G) can be patterned to improve penetration of the incident light to the active layer of the device, preferably by minimizing reflection losses.

Electrodes (B) and (F) of a solar cell according to Fig. 2 are formed of an organic, inorganic or hybrid material with sufficient electrical conductivity. As referred to herein, sufficient electrical conductivity is reached for a bulk material at the level of at least 500 S/m (e.g. 2000 S/m or 100000 S/m). For the thin films, a surface resistance below 200 Ohm, preferably 15 Ohm, has to be reached to enable sufficient electrical conductivity. Exemplary inorganic materials are represented by metals, preferably silver, aluminium, platinum, gold, nickel, metal alloys, preferably steel or nickel-chrome and conductive metal oxides preferably doped tin oxide, zinc oxide and etc.. Organic conductors are represented by organic meterials, preferably ionic salts formed by tetrathiafulvalenes and tetracyanoquinodimethane, doped conjugated polymers, preferably doped PEDOT or doped polyaniline or doped small molecules, preferably doped aromatic amines. Hybrid conducting materials are represented by metal grids coated with organic conducting materials.

A photovoltaic cell according to the present invention, preferably an organic photovoltaic cell according to Fig. 2, might preferably include a hole-selective buffer layer (C), which blocks the electrons and non-dissociated excitations and conducts the holes. The hole-selective buffer layer is generally formed from a material which, at the thickness used in the photovoltaic cell, can block efficiently electrons and non-dissociated excitons and transport the positive charge carriers (holes) to the respective electrode. Preferably a hole-selective buffer layer is composed of organic materials, preferably aromatic polyamines, conjugated polymers preferably polyaniline or polythiophenes in a pristine or doped state; inorganic materials, preferably metal oxides very preferred MoO₃, V₂O₅, metal chalcogenides, preferably Ag₂S, CuS and other types of compounds preferably salts, especially preferred CsHSO₄; or hybrid materials represented by blends or layer-by-layer assembles of the aforementioned organic and inorganic materials.

A photovoltaic cell according to the present invention, preferably an organic photovoltaic cell according to Fig. 2, might preferably include an electron-selective buffer layer (E), which blocks the holes and non-dissociated excitations and conducts the electrons. The electron-selective buffer layer (E) is generally formed from a material which, at the thickness used in the photovoltaic cell, can block holes and non-dissociated excitons efficiently and transport the negative charge carriers (electrons) to the respective electrode. An exemplary electron-selective buffer layer is composed of organic materials preferably quinones, halogenated phthalocyanines, nitrogen-based heterocycles, preferably bathocuproine, fullerenes and their derivatives, metal complexes, preferably AlQ₃ which is (Tris(8-hydroxyquinoline)aluminium [CAS No 2085-33-8], and etc, inorganic materials preferably metal chalcogenides, preferably Sb₂S₃, ZnS, ZnSe, CdS, metal oxides, preferably ZnO, TiO₂, MgO, hydroxides preferably NaOH, salts, preferably Na₂CO₃, Cs₂CO₃, LiF, thin metal coatings preferably Al, Ca, Ba, Sr or hybrid materials represented by blends or layer-by-layer assembles of the aforementioned organic and inorganic materials.

In some embodiments of the present invention the hole selective and electron selective buffer layers might contain carbon-based materials such as graphene, graphene oxide, carbon nanotubes, amorphous carbons and etc.

The performance of a photovoltaic device is characterized by the short circuit current, open circuit voltage, fill factor and energy or power conversion efficiency. The term fill factor, as used in the present invention, refers to the ratio of the maximal electrical power produced by the device (Vₘₚ x Iₘₚ) divided by the short circuit current density (I_{SC}) and open circuit voltage (V_{OC}) in light-on current density - voltage characteristics of solar cells. The term short circuit current density (I_{SC}), as used herein, corresponds to the maximal current measured through the load under short-circuit conditions. The term open circuit voltage (V_{OC}), as used herein, is the maximal voltage obtainable at the load under open-circuit conditions. The term power (or energy) conversion efficiency (PCE), as used herein, is the ratio of electrical power output from a device to the light power input (Pᵢₙ) defined as PCE = V_{OC} x I_{SC} x FF under 1 sun (100 mW/cm²) illumination conditions.

The implementation of the fulleropyrrolidines of formula (1) improves open circuit voltage (V_{OC}) and overall energy conversion efficiency (PCE) of organic photovoltaic devices.

The present invention is directed to the use of fulleropyrrolidines of the formula (1): 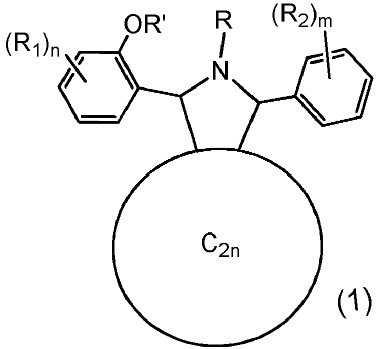 wherein
C₂ₙ is a carbon cage of C₆₀ or C₇₀ fullerene wherein,
R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group.
R₁ and R₂ are the same or independent from each other and represent a substitutent selected from the group of a hydrogen atom, an optionally substituted C₁-C₂₀ alkyl group, a C₁-C₂₀ haloalkyl group, preferably a C₁-C₂₀ fluoroalkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group, while indexes n and m represent the number of substituents R₁ and R₂ in the corresponding phenyl rings attached to the pyrrolidine unit and are independent from each other an integer from 1 to 3, to improve open circuit voltage (V_{OC}) and overall energy conversion efficiency (PCE) of organic photovoltaic devices and to enhance the power conversion efficiency of solar cells, preferably of organic solar cells.

The present invention is additionally directed to a process for the enhancement of the power conversion efficiency of solar cells, preferably of organic solar cells, by applying fulleropyrrolidines of the formula (1) 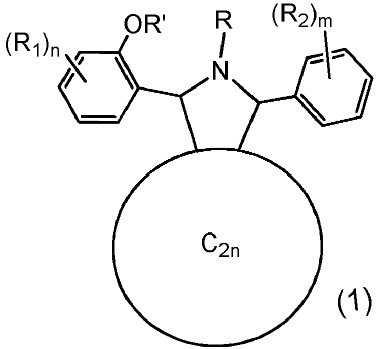 wherein
C₂ₙ is a carbon cage of C₆₀ or C₇₀ fullerene wherein,
R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group,
R₁ and R₂ are the same or independent from each other and represent a substitutent selected from the group of a hydrogen atom, an optionally substituted C₁-C₂₀ alkyl group, a C₁-C₂₀ haloalkyl group, preferably a C₁-C₂₀ fluoroalkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group,
while indexes n and m represent the number of substituents R₁ and R₂ in the corresponding phenyl rings attached to the pyrrolidine unit and are independent from each other an integer from 1 to 3, to the photoactive layer of such organic solar cell, preferably to the photoactive layer (D) of a solar cell according to Fig. 2.

It will be understood that the specification and examples are illustrative but not limitative of the present invention and that other embodiments within the spirit and scope of the invention will suggest themselves to those skilled in the art.

### Examples:

### Example 1 Synthesis of fulleropyrrolidine (1i)

The method was based on a general description of M. Maggini et al. J. Am. Chem. Soc. 1993. V. 115. P. 9798. A 250 mL three-necked round-bottom flask was equipped with reversed condenser, thermometer and magnetic stirrer. Fullerene C₆₀ (720 mg, 1 mmol) and 1,2-dichlorobenzene (150 mL) were added. The reaction system was evacuated three times and filled with argon. When the [60]fullerene got dissolved under stirring at room temperature, 440 mg of 2-((2-ethylhexyl)amino)-2-(2-methoxyphenyl)acetic acid (1.5 mmol) and 255 mg of 2,4,6-trimethoxybenzaldehyde (1.3 mmol) was added in one portion in the stream of argon. The reaction mixture was heated under stirring at 120-130 °C for 3 hours. The reaction mixture was concentrated to dryness at the rotary evaporator. The oily residue was redissolved in toluene (50 ml) and then hexane (200 ml) was added. The mixture was filtered and the filtrate was purified by column chromatography on silica using mixtures of toluene with hexane as eluent. The title product was eluted with toluene-hexane 1:1 v/v mixture. The solution of (1i) was concentrated at the rotary evaporator to the volume of ca. 20 mL and then 80 mL of methanol was added. The precipitated product was collected by centrifugation and dried in vacuum. The yield of (1i) was 42% based on C₆₀ used for reaction. Compounds (1f), (1g), (1h), (1j) and (1k) were prepared in a similar way.

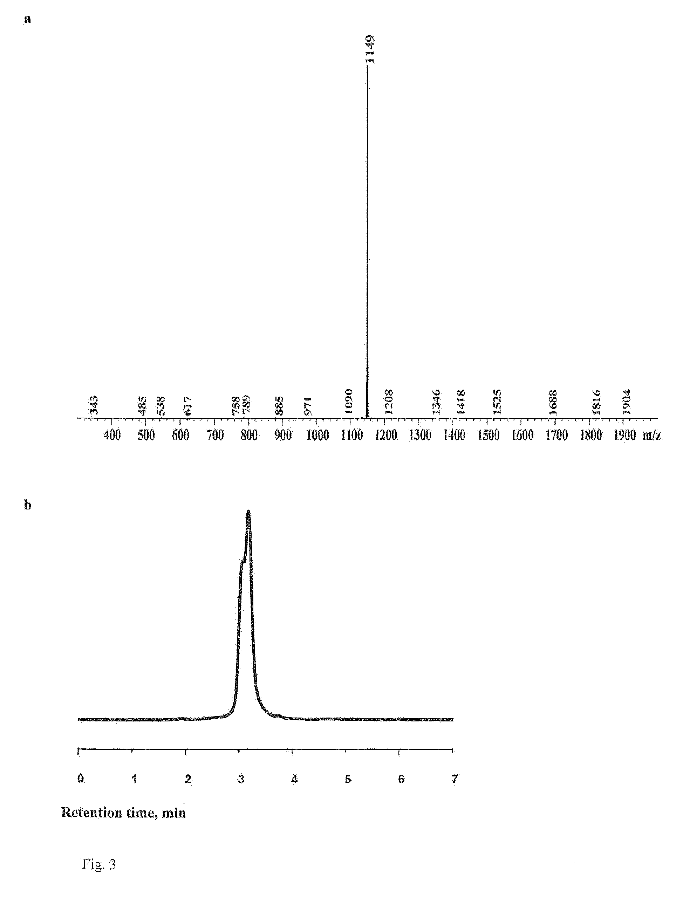

Fig. 3a demonstrates the ESI mass spectrum of (1i) (70% toluene + 30% of methanol, v/v) which shows m/z = 1149 corresponding to [M]⁻.
Fig. 3b demonstrates the HPLC profile for (1i) (reversed phase C₁₈, Phenomenex® Luna 5u C18 (2) 100A, 4.6x150 mm column, toluene/acetonitrile 40:60 v/v eluent, 1 mL/min flow rate) which shows the presence of two badly resolved peaks due to two stereoisomers.

### Example 2 Solar cells comprising fulleropyrrolidines of general formula (1) as electron acceptor component and P3HT as an electron donor polymer

A photovoltaic cell, according to the present invention, as illustrated in Fig. 2, was constructed in the following way. The patterned ITO-coated glass substrates (25 x 25 mm) were sonicated consecutively with acetone, isopropyl alcohol, and deionized water for 10 min. Subsequently, thin calcium layer (3 nm) was evaporated in vacuum on the top of ITO-coated glass substrates. For the active layer deposition, a blend of 9 mg of the fulleropyrrolidine (compound of formula (1) or some reference compound) and 12 mg of P3HT, both dissolved in 1 ml of chlorobenzene, was spin-coated at the spinning frequency of 900 rpm. The resulting films were annealed at 155°C for 3 min and then the top electrode was deposited in high vacuum (10⁻⁶ mbar) by sequential evaporation of 5 nm of molybdenium trioxide and 100 nm of Ag. The device could have been encapsulated using appropriate barrier foils and sealing adhesive materials (to form top substrate G as shown in Fig. 2).

To illustrate the improved performance of the photovoltaic cells according to the present inventtion, the current density - voltage (I-V) characteristics were examined for the devices comprising the fulleropyrrolidines of general formula (1) and for the devices comprising reference [60]-PCBM as electron acceptor component.

The obtained solar cell parameters are listed in Table 3. Standard characteristics of photovoltaic cells (e.g. open circuit voltage V_{OC}, short circuit current I_{SC}, fill factor FF, energy (power) conversion efficiency PCE) are given in Table 1 along with two additional parameters: ΔV_{OC} and ΔPCE. The parameter ΔV_{OC} was calculated as V_{OC}(P3HT/fullerene derivative)-V_{OC}(P3HT/PCBM) and represents the improvement in the open circuit voltage of the solar cell compared to the reference P3HT/PCBM device. The parameter ΔPCE was calculated as PCE(P3HT/fullerene derivative)-PCE(P3HT/PCBM) and represents the improvement in the overall power (energy) conversion efficiency of the solar cell compared to the reference P3HT/PCBM device.

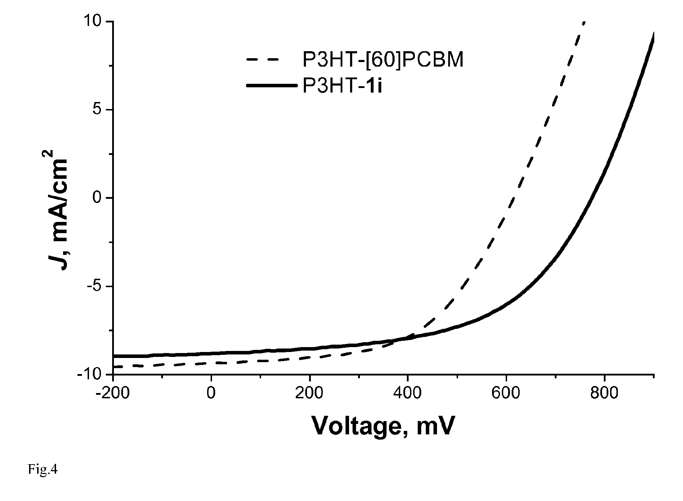

Fig. 4 demonstrates the I-V curves of the solar cells based on (1i)/P3HT and reference [60]PCBM/P3HT composites under simulated AM1.5 conditions. A strong increase in the open circuit voltage for the (1i)/P3HT system compared to the reference [60]PCBM/P3HT blend is evident from the comparison of these I-V curves.

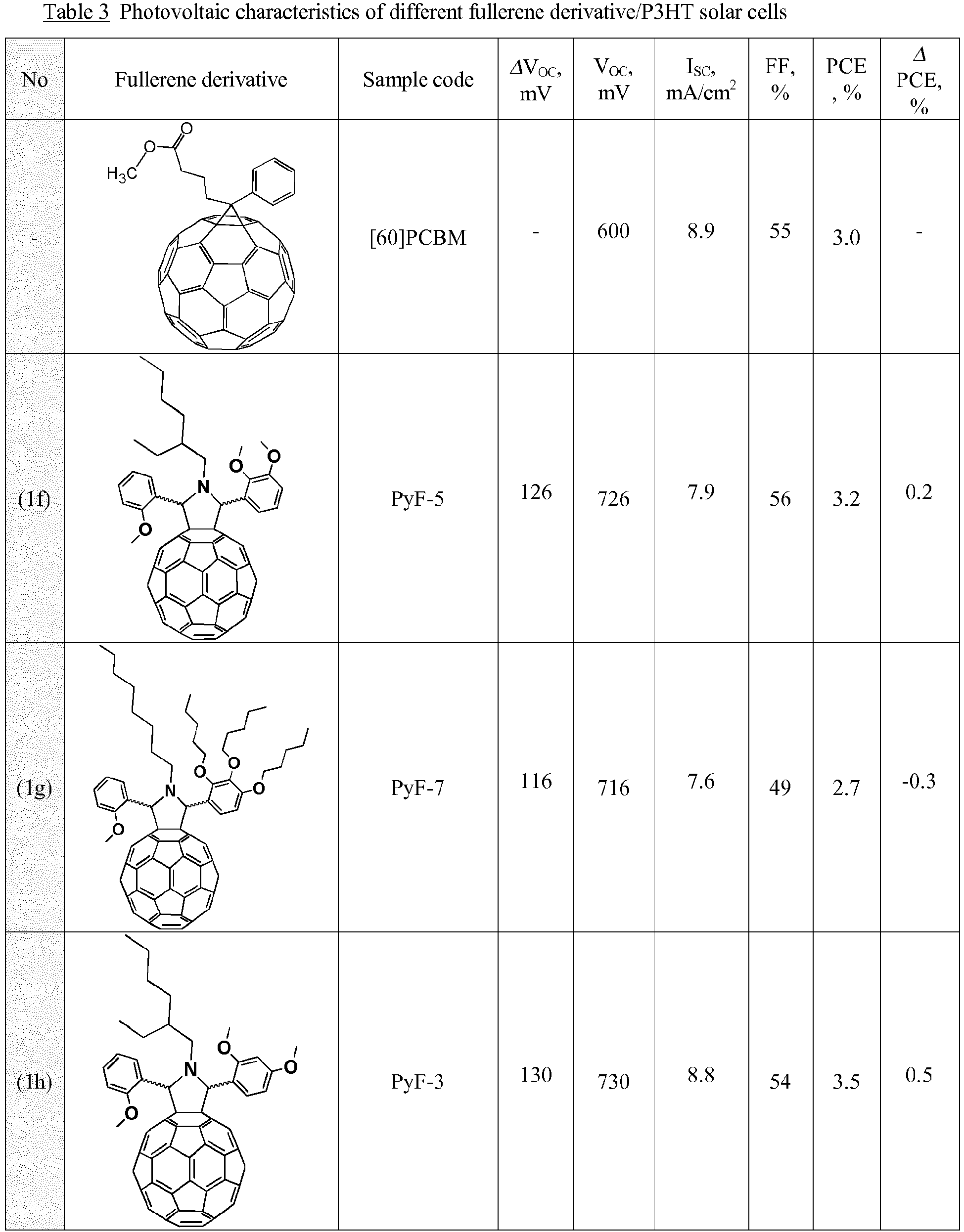

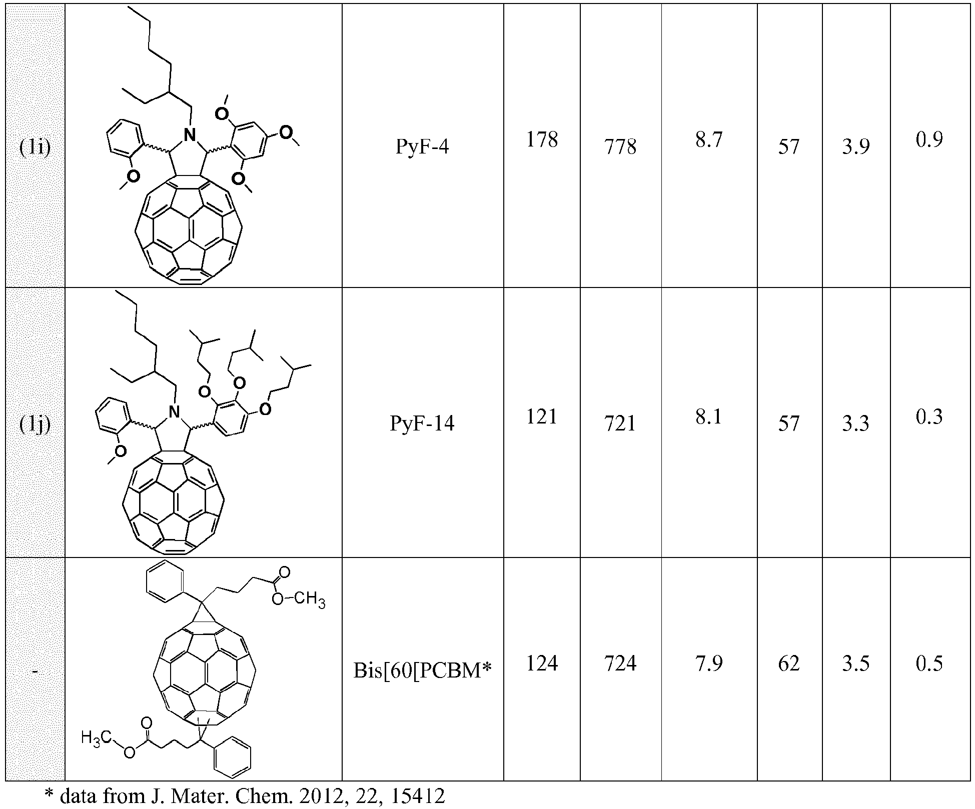

Considering the data presented in Table 3 one can notice that the devices comprising fulleropyrrolidine derivatives of the general formula (1) show significantly improved open circuit voltages (ΔV_{OC} from 116 to 178 mV) and improved power conversion efficiencies (ΔPCE=0.9% for the best system) compared to the reference P3HT/PCBM device. Moreover, the fulleropyrrolidines according to the present invention outperformed the bis-functionalized fullerene derivative bis-[60]PCBM in terms of open circuit voltage (fulleropyrrolidines (1f), (1h) and (1i) and solar cell power conversion efficiency (fulleropyrrolidine (1i)). Moreover, compounds (1h) and (1i) outperformed in terms of open circuit voltage and PCE all other fulleropyrrolidine-based acceptor materials known by today.

It should be noted also that the best-performing fulleropyrrolidine (1i) improves the open circuit voltage and the power (energy) conversion efficiency of the device by 30% relative to the P3HT/ PCBM reference system.

## Claims

1. Fulleropyrrolidines of the formula (1) 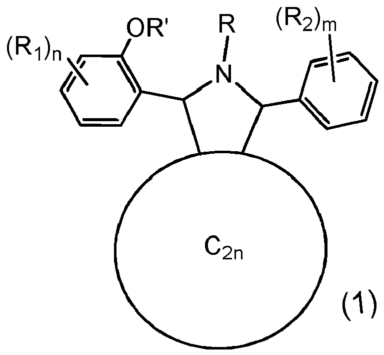 wherein
C₂ₙ is a carbon cage of C₆₀ or C₇₀ fullerene wherein,
R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group,
R₁ and R₂ are the same or independent from each other and represent a substitutent selected from the group of a hydrogen atom, an optionally substituted C₁-C₂₀ alkyl group, a C₁-C₂₀ haloalkyl group, preferably a C₁-C₂₀ fluoroalkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group,
while n and m represent the number of substituents R₁ and R₂ in the corresponding phenyl rings attached to the pyrrolidine unit and are independent from each other an integer from 1 to 3.

2. Fulleropyrrolidines according to Claim 1, **characterized in that** R and R' are the same or independent from each other and represent a substitutent selected from the group of an optionally substituted C₁-C₂₀ alkyl group, a C₁-C₂₀ haloalkyl group, a C₁-C₂₀ alkylether group, a aryl-C₁-C₂₀ alkyl group or a heteroaryl-C₁-C₂₀ alkyl group and R₁ and R₂ are the same or independent from each other they represent a C₁-C₂₀ alkoxy group, a C₁-C₂₀ fluoroalkoxy group, a C₁-C₂₀ alkylthio group or a C₁-C₂₀ fluoroalkylthio group, while n = 1 or 2 and m = 1, 2 or 3.

3. Fulleropyrrolidines according to Claim 1, **characterized in that** R and R' are the same or independent from each other and represent a substitutent selected from the group of an optionally substituted C₁-C₂₀ alkyl group, a C₁-C₂₀ haloalkyl group, a C₁-C₂₀ alkylether group, a aryl-C₁-C₂₀ alkyl group or a heteroaryl-C₁-C₂₀ alkyl group and R₁ and R₂ are the same or independent from each other and represent C₁-C₂₀ alkoxy group or C₁-C₂₀ alkylthio group, while n = 1 or 2 and m = 1, 2 or 3.

4. Fulleropyrrolidines according to Claim 1, **characterized in that** R and R' are the same or independent from each other and represent a substitutent selected from the group of an optionally substituted C₁-C₂₀ alkyl group, a C₁-C₂₀ haloalkyl group, a C₁-C₂₀ alkylether group, a aryl-C₁-C₂₀ alkyl group or a heteroaryl-C₁-C₂₀ alkyl group and R₁ and R₂ are the same or independent from each other and represent a C₁-C₂₀ alkoxy group or a C₁-C₂₀ alkylthio group, while n = 2 and m = 3.

5. Fulleropyrrolidines according to any of Claims 1 to 4, **characterized in that** R represents a substitutent selected from the group of an optionally substituted C₁-C₂₀ alkyl group or a C₁-C₂₀ alkylether group.

6. Fulleropyrrolidines according to any of Claims 1 to 5, **characterized in that** R' represents a substitutent selected from the group of an optionally substituted C₁-C₂₀ alkyl group or a C₁-C₂₀ alkylether group.

7. Fulleropyrrolidines according to any of Claims 1 to 6, **characterized in that** R₁ and R₂ are the same or independent from each other and represent a substitutent selected from the group of a hydrogen atom, a C₁-C₂₀ alkoxy group or a C₁-C₂₀ alkylthio group.

8. Fulleropyrrolidines according to any of Claims 1 to 7, **characterized in that** R₁ and R₂ are the same or independent from each other and represent a hydrogen atom or a C₁-C₂₀ alkoxy group.

9. Fulleropyrrolidines according to any of Claims 1 to 8, **characterized in that** R₁ and R₂ are the same or independent from each other and represent a hydrogen atom or a C₁-C₂₀ alkylthio group.

10. Fulleropyrrolidines according to any of Claims 1 to 9 having one of the formulas a1-a6, b1-b6, c1-c6, d1-d6, e1-d6 or f1-f6: 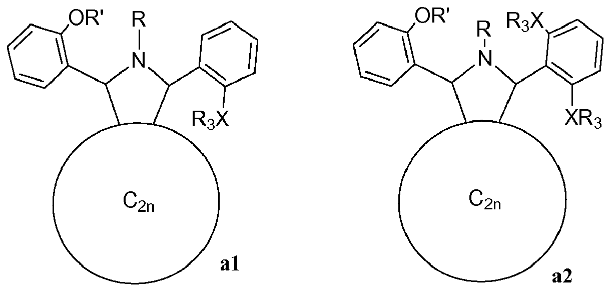 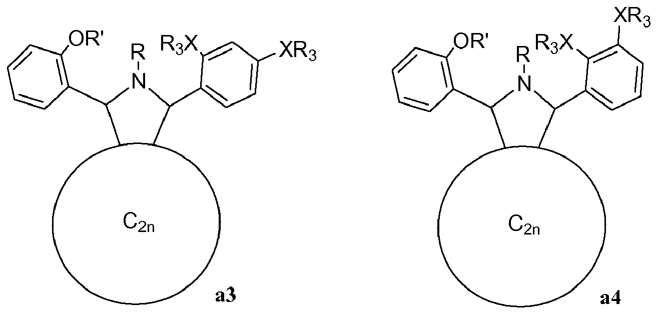 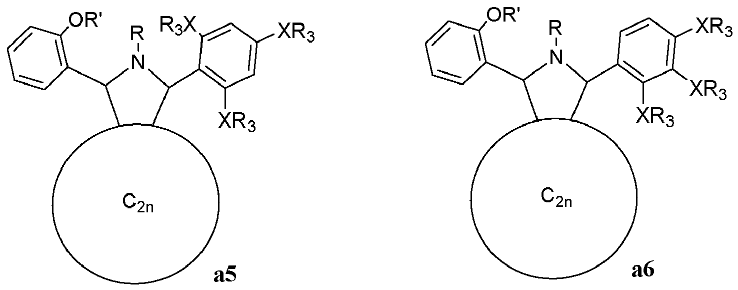 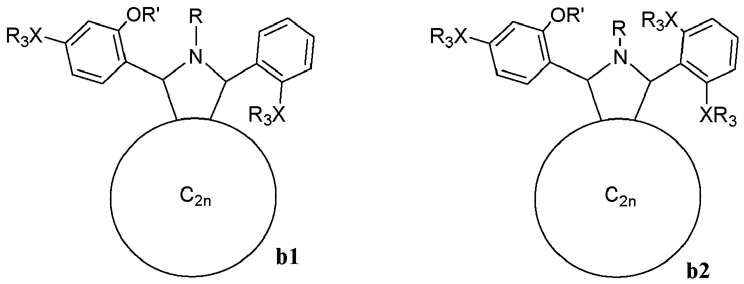 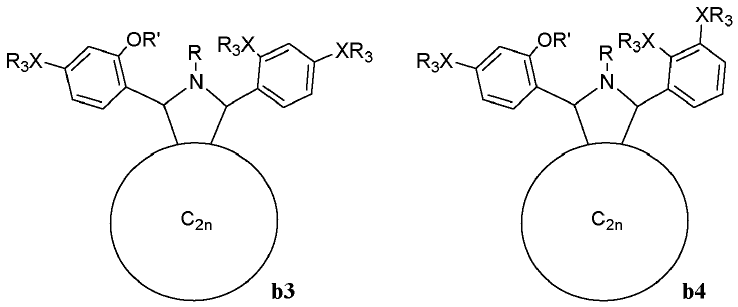 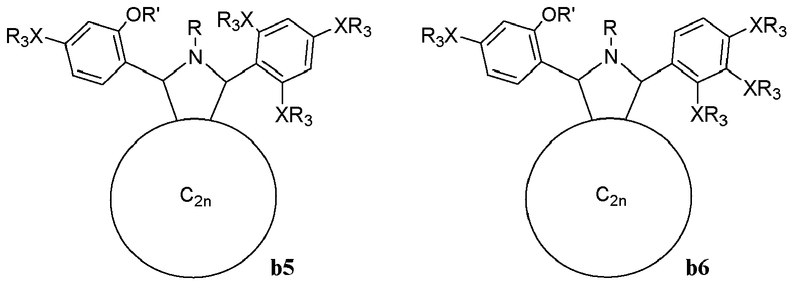 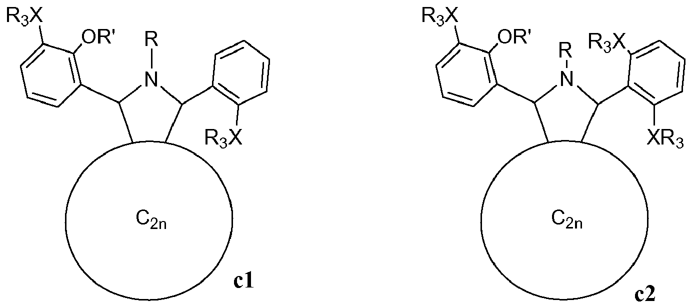 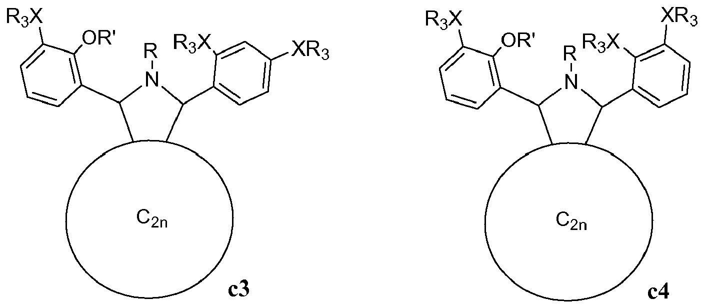 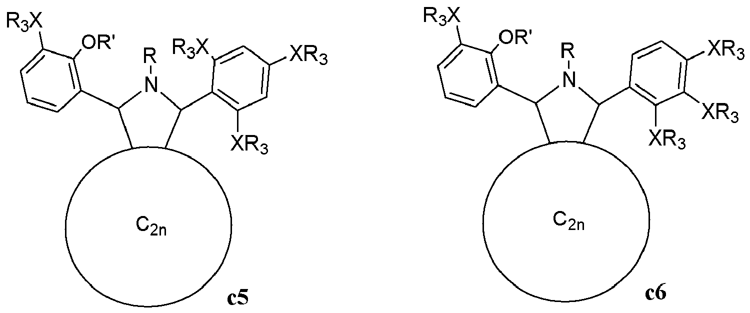 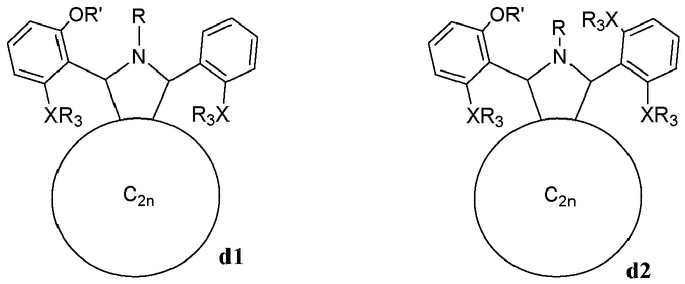 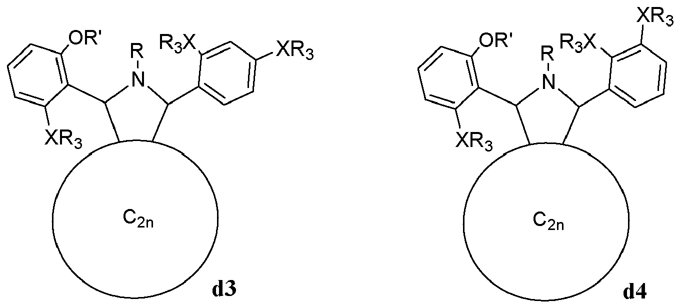 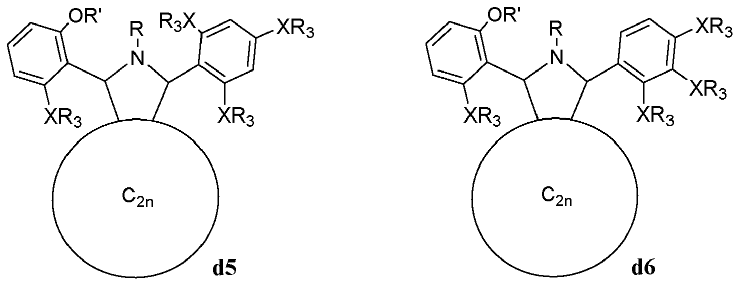 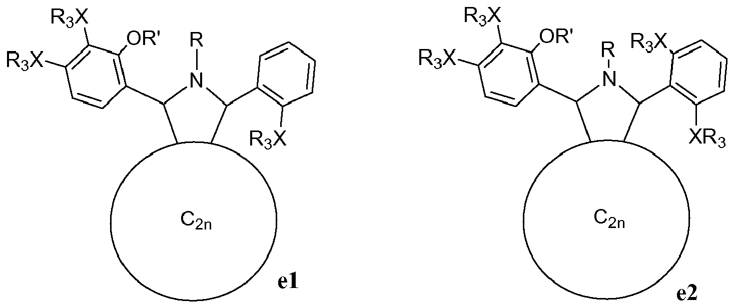 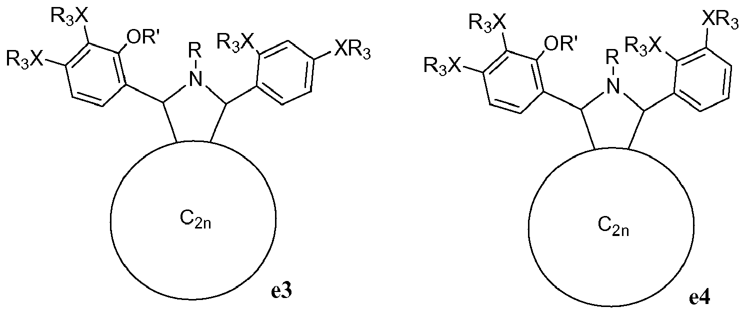 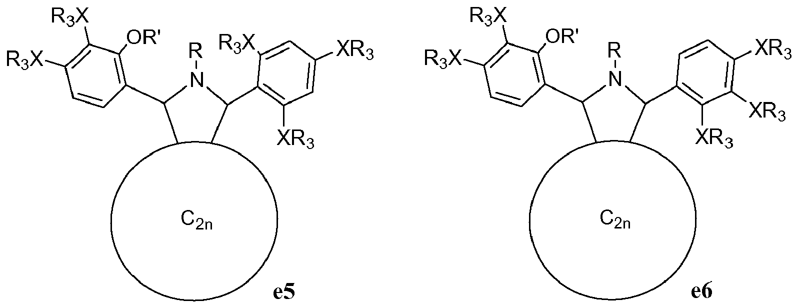 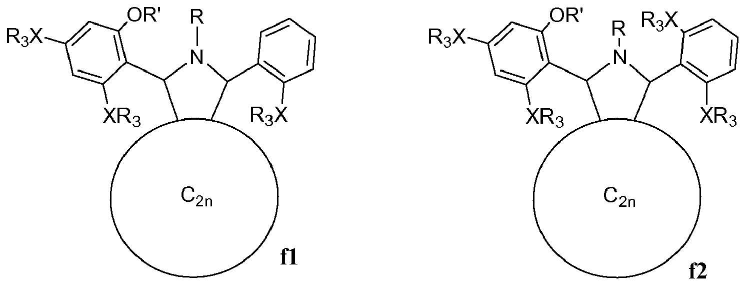 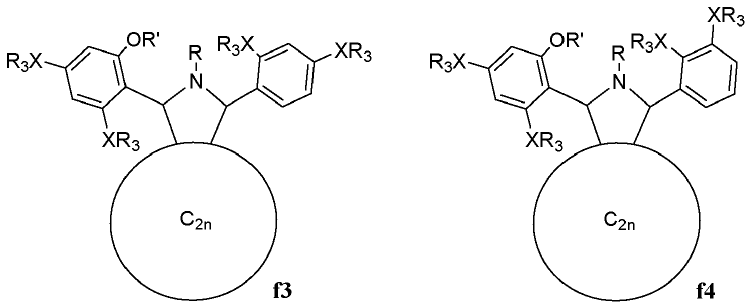 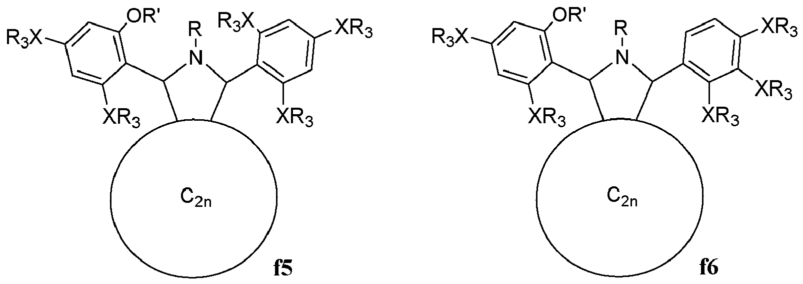 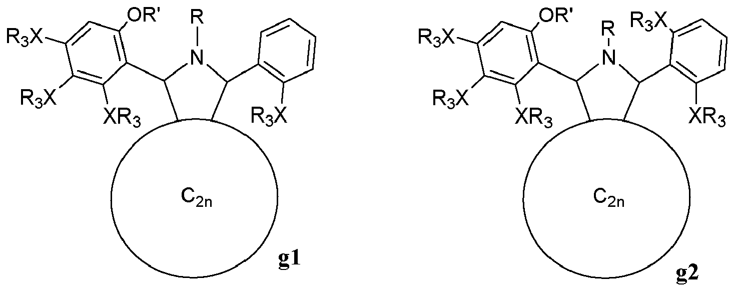 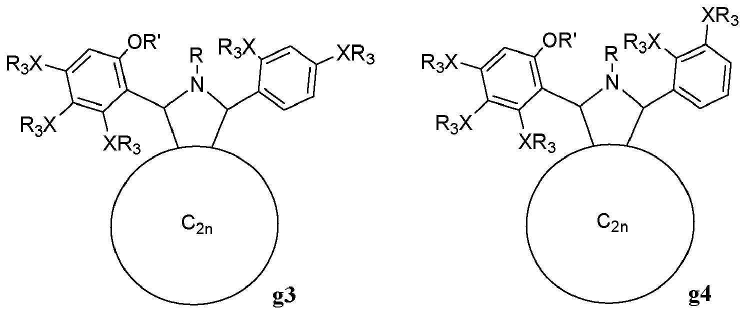 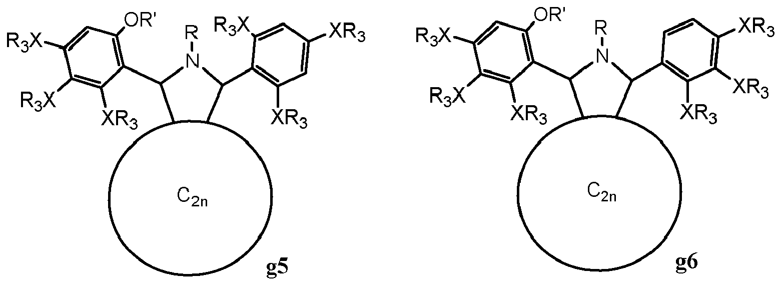
wherein C₂ₙ is a carbon cage of C₆₀ fullerene or C₇₀ fullerene, preferably C₆₀ fullerene;
X represents O or S;
R, R' and R₃ are the same or independent from each other and represent a substituent selected from the group of an optionally substituted C₁-C₂₀ alkyl group, a C₁-C₂₀ fluorinated alkyl group or a C₁-C₂₀ alkylether group.

11. A process for the manufacture of fulleropyrrolidines of formula (1) 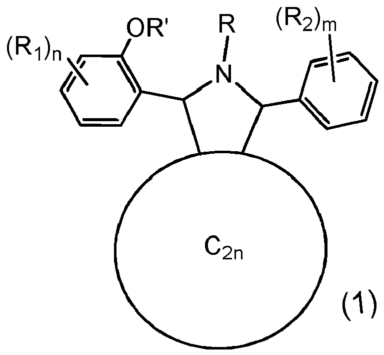 wherein
C₂ₙ is a carbon cage of C₆₀ or C₇₀ fullerene wherein,
R and R' are the same or independent from each other and represent a substitutent selected from the group of an optionally substituted C₁-C₂₀ alkyl group, a C₁-C₂₀ haloalkyl group, preferably a C₁-C₂₀ fluoroalkyl group, a C₁-C₂₀ alkylether group, a aryl-C₁-C₂₀ alkyl group or a heteroaryl-C₁-C₂₀ alkyl group,
R₁ and R₂ are the same or independent from each other and represent a substitutent selected from the group of a hydrogen atom, an optionally substituted C₁-C₂₀ alkyl group, a C₁-C₂₀ haloalkyl group, preferably a C₁-C₂₀ fluoroalkyl group, a C₁-C₂₀ alkylether group, a C₁-C₂₀ fluorinated alkylether group, a C₁-C₂₀ alkoxy group, a C₁-C₂₀ fluoroalkoxy group, a C₁-C₂₀ alkylthio group, a C₁-C₂₀ fluoroalkylthio group, a aryl-C₁-C₂₀ alkyl group or a heteroaryl-C₁-C₂₀ alkyl group,
while n and m represent the number of substituents R₁ and R₂ in the corresponding phenyl rings attached to the pyrrolidine unit and are independent from each other an integer from 1 to 3,
**characterized in that** such process is performed according to the scheme 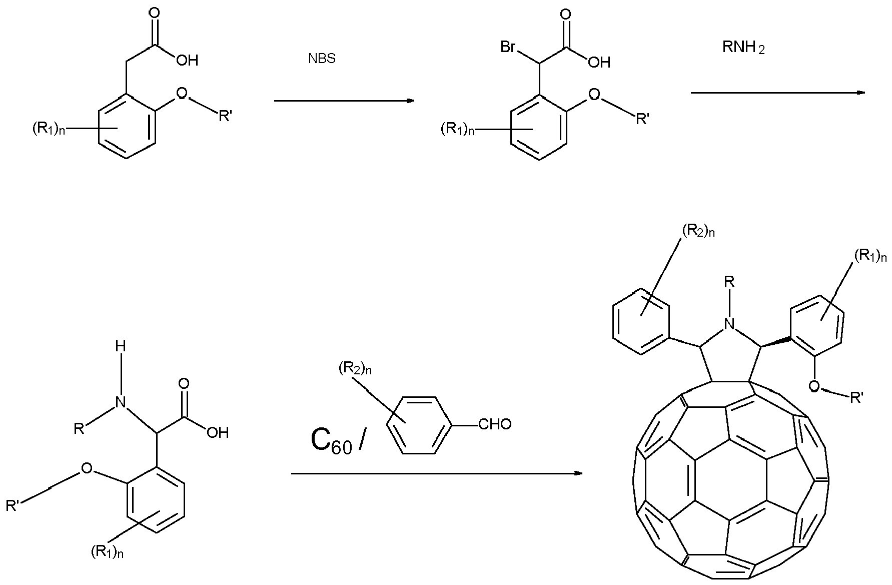 Wherein NBS represents 1-bromo-2,5-pyrrolidinedione and R, R',R₁,R₂, m and n have the meanings given for formula (1).

12. Use of fulleropyrrolidines according to any of Claims 1 to 10 in a thin film of an photovoltaic cell, preferably in a thin film of an organic photovoltaic cell or of an organic solar cell.

13. Use according to Claim 12, **characterized in that** at least one fulleropyrrolidine is used in combination with at least one electron donor component selected from (i) a fully conjugated or partially conjugated polymer (ii), a low molecular weight donor material or (iii) inorganic nanoparticles.

14. Use according to Claim 13, **characterized in that** the fully conjugated or partially conjugated polymer (i) is selected from the group of poly(3-hexylthiophene) P3HT, poly(2,7-(9,9-di(alkyl)-fluorene)-alt-5,5-(4',7'-di-2-thienyl-2', 1',3'-benzothiadiazole)) (PFDTBT), poly(2,6-(4,4-bis-(2'-ethylhexyl)-4H-cyclopenta(2,1-b;3,4-6')dithiophene)-alt-4',7'-(2',1',3'-benzothiadiazole) (PCPDTBT), poly(2,6-(4,4-di(n-dodecyl)-4H-cyclopenta(2,1-b;3,4,-6')dithiophene)alt-5,5-(4',7'-di-2-thienyl-2',1',3'-benzothiadiazole)) and poly[N-9'-heptadecanyl-2,7-carbazole-alt-5,5-(4',7'-di-2-thienyl-2',1',3'-benzothiadiazole) (PCDTBT), preferably poly(3-hexylthiophene).

15. Use according to any of Claims 12 to 14 to improve the open circuit voltage (V_{OC}) and overall energy conversion efficiency (PCE) of organic photovoltaic devices and to enhance the power conversion efficiency of solar cells, preferably of organic solar cells.
